# EUROPEAN PATENT APPLICATION

(11) **EP 0 825 198 A1**
(43) Date of publication of application: **25.02.1998**
(21) Application number: 97200130.9
(22) Date of filing: 17.01.1997
(51) Int. Cl.: C07K 14/47, C12Q 1/68

(54) **Plag gene family and tumorigenesis**

(30) Priority: 22.08.1996 EP 96202339
(71) Applicant: K.U. LEUVEN RESEARCH & DEVELOPMENT, 3000 Leuven (BE); Holdingbolaget vid Göteborgs Universitet AB, 413 90 Göteborg (SE)
(72) Inventor: Van de Ven, Willem Jan Marie, B-3000 Leuven (BE); Stenman, Karl Göran David, S-426 76 Västra Frölunda (SE); Kas, Koen Pieter, B-2110 Wijnegem (BE); Voz, Marianne Léontine, B-4300 Waremme (BE)
(74) Representative: Land, Addick Adrianus Gosling

(57) **Abstract**

The present invention relates to a novel gene family identified as T-genes and being implicated in tumor-genesis having the nucleotide sequence of any one of the strands of any one of the members of the PLAG and CTNNB1 gene families. The genes and their derivatives may be used in various diagnostic and therapeutic applications.

## Description

The present invention relates to the identification of the PLAG gene family as a family of genes frequently associated with tumorigenesis. The invention in particular relates to the identification of one member of this gene family that is involved in benign tumors such as those with chromosome anomalies involving a particular region of the long arm of chromosome 8, for instance but not restricted to pleomorphic adenomas of the salivary glands with involvement of chromosome 8q12 and a translocation partner chromosome, very frequently chromosome 3 [i.e. t(3;8)(p21;q12)]. In addition, the invention relates to another member of this novel family as the gene whose expression is frequently abrogated in malignant tumors such as for example but not restricted to malignant salivary gland tumors. Furthermore, the invention concerns the identification of the CTNNB1 gene as a prototype of tumor-specific breakpoint region genes and frequent fusion partner of the PLAG genes. The invention relates in particular to the use of the members of the PLAG gene family and corresponding fusion partner genes as well as their derivatives in diagnosis and therapy.

Multiple independent cytogenetic studies have firmly implicated chromosome region 8q11-13 in the following tumor types: pleomorphic adenomas of the salivary glands (high frequency), pleomorphic adenomas of the lacrimal glands (high frequency), lipoblastomas (high frequency), solitary lipomas (low frequency), rhabdomyosarcomas (low frequency), and renal cell carcinomas (low frequency). As an illustration, we here focus on pleomorphic adenomas of the salivary gland. This type of tumor constitutes a benign epithelial tumor that originates from the major and minor salivary glands. It is the most common type of salivary gland tumor and accounts for almost 50% of all neoplasms in these organs; 85% of the tumors are found in the parotid gland, 10% in the minor salivary glands and 5% in the submandibular gland. About 50% of these adenomas appear to have a normal karyotype but cytogenetic studies have also revealed recurrent specific chromosome anomalies. Frequently observed anomalies include aberrations of chromosome 8, usually involving the 8q12 region with as most common aberration a t(3;8)(p21;q12), and aberrations of chromosome 12, usually translocations involving region 12q13-q15. Non-recurrent clonal chromosome abnormalities have also been reported. The highly specific pattern of chromosome rearrangements with consistent breakpoints at 8q12 and 12q13-q15 suggests that these chromosomal regions harbour genes that might be implicated in the development of these tumors.

In previous physical mapping studies, the chromosome 8q12 breakpoint in pleomorphic adenoma of the salivary glands was mapped between MOS and PENK. In an effort to molecularly clone the gene affected by the chromosome 8q12 aberrations in the various tumors, the present inventors chose directional chromosome walking as a structural approach to define the DNA region encompassing these breakpoints.

As a starting point for chromosome walking, the MOS locus was used. During these walking studies, YAC clones corresponding to eight different loci in 8q11-12 were isolated and mapped by FISH analysis. Initially, the breakpoints were mapped within a 1 Mb region flanked by MOS proximally and by the genetic marker DSS166 distally. One YAC (CEPH 166F4) within this region was shown to span the t(3;8) breakpoint in two tumors. Subsequently, it was shown by FISH that the chromosomal breakpoints as present in a number of primary pleomorphic adenomas were clustered within a small chromosomal segment which has been designated pleomorphic adenoma breakpoint cluster region (PA-BCR).

It has thus been found that essentially all breakpoints of chromosome 8q12 map in a region indicated herein as PA-BCR. Further research revealed that in this region a member of the PLAG gene family, the PLAG1 gene, can be identified as the breakpoint target gene and postulated tumor aberrant growth gene. The PLAG gene family is a subclass of the zinc finger gene superfamily. The CTNNB1 gene was identified as the fusion partner gene of PLAG1. Molecular analysis of the chromosome breakpoints revealed that the chromosome translocations resulted in gene fusions. Fusions appeared to occur in the 5'-non-coding regions of both genes (PLAG1 and CTNNB1), exchanging regulatory control elements while preserving the coding sequences. Due to the t(3;8)(p21;q12), PLAG1 is activated and expression of CTNNB1 is down-regulated. Activation of PLAG1 was also observed in adenomas with variant translocations, i.e. t(5;8) and t(8;15). The results of the present inventors indicate that PLAG1 activation due to promoter swapping is a crucial event in salivary gland tumorigenesis.

The preferential fusion partner of PLAG1 is the CTNNB1 gene, which encodes β-catenin, a cytoplasmic protein of about 88 kD. Its frequent involvement in pleomorphic adenomas point towards a critical role of the CTNNB1 gene. The major role of CTNNB1 may be to provide an active promoter in front of the PLAG1 gene. However, since the observed promoter swapping between PLAG1 and CTNNB1 leads to down-regulation of CTNNB1 expression, it may also lead to other physiological consequences, especially since β-catenin is a protein that has been implicated in highly diverse processes, such as human colon cancer, epithelial cell adhesion, embryonal axis formation in Xenopus, and pattern formation in Drosophila.

According to the present invention the aberrations in the PLAG1 gene on chromosome 8 and the CTNNB1 gene on chromosome 3 have been used as a model to reveal the more general concept of the involvement of members of these gene families in tumorigenesis. Although both gene families are known per se, up till the present invention the correlation between these families and tumor inducing chromosome aberrations, like translocations, deletions, insertions and inversions, has not been anticipated. Furthermore, until now, it was not previously demonstrated that alterations in the physiological expression level of the members of the gene family are probably also implicated in tumor development. According to the invention, it was demonstrated that in normal adult cells the expression level of the PLAG1 gene is practically undetectable, whereas in aberrantly growing cells, the expression level is significantly increased. It appears that PLAG1 is a oncogene.

The research that led to the present invention indicates that it is probable that abberations in the PLAG1 gene, either in its sequence and/or its expression, will usually lead to a benign tumor. Furthermore, preliminary research indicated that expression of PLAG1 is increased in various types of malignant salivary gland tumors.

Another member of the PLAG gene family is the PLAG2 gene. The present inventors have identified this gene, determined its nucleotide sequence and predicted its amino acid sequence. It was found that PLAG2 mapped to a region frequently deleted in malignant salivary gland tumors (6q24). Thus, contrary to PLAG1, PLAG2 may be a tumorsuppressor gene.

The present invention now provides for a tool to investigate these theories and may ultimately lead to a means for distinguishing between benign and malignant tumors. This knowledge can then lead to more efficient methods of treatment. Thus, the present invention now provides for the members of the gene families or derivatives thereof in isolated form and their use in diagnostic and therapeutic applications. Furthermore, the knowledge on the location and nucleotide sequence of the genes may be used to study their rearrangements or expression and to identify a possible increase or decrease in their expression level and the effects thereof on cell growth. Based on this information diagnostic tests or therapeutic treatments may be designed.

In this application, "PLAG" will be used to indicate the involvement of these types of genes in various types of tumors, not necessarily restricted to pleomorphic adenomas of the salivary glands. The term refers to all members of the PLAG gene family involved in non-physiological proliferative growth, and in particular involved in benign or malignant tumors. Members of the PLAG gene family show homology between zinc finger domains that are typical for the PLAG1 gene. However, according to the invention it was furthermore found that even breaks outside the actual genes but in the vicinity thereof might result in aberrant growth. The term "PLAG gene" is therefore also intended to include the immediate vicinity of the gene. The skilled person will understand that the "immediate vicinity" should be understood to include the surroundings of the gene in which the breaks or mutations will result in the above-defined non-physiological proliferative growth.

The term "Tumorigenesis gene" or "T-gene" will be used to indicate all members of this novel PLAG gene family and their corresponding translocation or fusion partners, like CTNNB1.

The term "wildtype cell" is used to indicate the cell not harbouring an aberrant chromosome. "Wildtype" or "normal" chromosome refers to a non-aberrant chromosome.

The present invention provides for various diagnostic and therapeutic applications that are based on the information that may be derived from the genes. This information not only encompasses its nucleotide sequence or the amino acod sequence of the gene product derived from the gene, but also involves the levels of transcription or translation of the gene.

The invention is thus two-fold. On the one hand the aberration in cell growth may be directly or indirectly caused by the physical breaks that occur in the gene or its vicinity. On the other hand the aberration in cell growth may be caused by a non-physiological expression level of the gene. This non-physiological expression level may be caused by the break, or may be due to another stimulus that activates or deactivate the gene. At present, the exact mechanism or origin of the aberrant cell growth is not yet completely unraveled. However, exact knowledge at this time is not necessary to define methods of diagnosis or treatment.

Diagnostic methods according to the invention are thus based on the fact that an aberration in a chromosome results in a detectable alteration in the chromosomes' appearance or biochemical behaviour. A translocation, for example will result in a first part of the chromosome (and consequently of a PLAG gene) having been substituted for another (second) part (further referred to as "first and second substitution parts"). The first part will often appear someplace else on another chromosome from which the second part originates. As a consequence hybrids will be formed between the remaining parts of both (or in cases of triple translocations, even more) chromosomes and the substitution parts provided by their translocation partners. Since it has now been found that the breaks occur in a PLAG gene, this will result in hybrid gene products of that PLAG gene. Markers, such as hybridising molecules like RNA, DNA or DNA/RNA hybrids, or antibodies will be able to detect such hybrids, both on the DNA level, and on the RNA or protein level.

For example, the transcript of a hybrid will still comprise the region provided by the remaining part of the gene/chromosome but will miss the region provided by the substitution part that has been translocated. In the case of inversions, deletions and insertions the gene may be equally afflicted.

Translocations are usually also cytogenetically detectable. The other aberrations are more difficult to find because they are often not visible on a cytogenetical level. The invention now provides possibilities for diagnosing all these types of chromosomal aberrations.

In translocations markers or probes based on the PLAG gene for the remaining and substitution parts of a chromosome in situ detect the remaining part on the original chromosome but the substitution part on another, the translocation partner.

In the case of inversions for example, two probes will hybridise at a specific distance in the wildtype gene. This distance might however change due to an inversion. In situ such inversion may thus be visualized by labeling a set of suitable probes with the same or different detectable markers, such as fluorescent labels. Deletions and insertions may be detected in a similar manner.

According to the invention the above in situ applications can very advantageously be performed by using FISH techniques. The markers are e.g. two cosmids one of which comprises exon 1 and the upstream region of the PLAG gene, while the other comprises the last exon and its downstream region. Both cosmids are labeled with different fluorescent markers, e.g. blue and yellow. The normal chromosome will show a combination of both labels, thus giving a green signal, while the translocation is visible as a blue signal on the remaining part of one chromosome (e.g. 8) while the yellow signal is found on another chromosome comprising the substitution part. In case the same labels are used for both probes, the intensity of the signal on the normal chromosome will be 100%, while the signal on the aberrant chromosomes is 50%. In the case of inversions one of the signals shifts from one place on the normal chromosome to another on the aberrant one.

In the above applications a reference must be included for comparison. Usually only one of the two chromosomes is afflicted. It will thus be very convenient to use the normal chromosome as an internal reference. Furthermore it is important to select one of the markers on the remaining or unchanging part of the chromosome and the other on the substitution or inverted part. In the case of the PLAG1 gene of chromosome 8, breaks are usually found in the 5'-UTR as is shown by the present invention. Probes based on the 5'- and 3'-UTR are thus very useful. As an alternative a combination of probes based on both translocation or fusion partners may be used, e.g. 5'-UTR of PLAG1 and 3'-UTR of CTNNB1.

"Probes" as used herein should be widely interpreted and include but are not limited to linear DNA or RNA strands, Yeast Artificial Chromosomes (YACs), or circular DNA forms, such as plasmids, phages, cosmids etc..

These in situ methods may be used on metaphase and interphase chromosomes.

Besides the above described in situ methods various diagnostic techniques may be performed on a more biochemical level, for example based on alterations in the DNA, RNA or protein or on changes in the physiological expression level of the gene.

Basis for the methods that are based on alterations in the chromosome's biochemical behavior is the fact that by choosing suitable probes, variations in the length or composition in the gene, transcript or protein may be detected on a gel or blot. Variations in length are visible because the normal gene, transcript(s) or protein(s) will appear in another place on the gel or blot then the aberrant one(s). In case of a translocation more than the normal number of spots will appear.

Based on the above principles the invention thus relates to a method of diagnosing cells having a non-physiological proliferative capacity, comprising the steps of taking a biopsy of the cells to be diagnosed, isolating a suitable PLAG gene-related macromolecule therefrom, and analysing the macromolecule thus obtained by comparison with a reference molecule originating from cells not showing a non-physiological proliferative capacity, preferably from the same individual. The PLAG gene-related macromolecule may thus be a DNA, an RNA or a protein. The PLAG gene may be either a member of the PLAG1 family or of the translocation partner gene family of which the CTNNB1 gene is the prototype.

In a specific embodiment the diagnostic method of the invention comprises the steps of taking a biopsy of the cells to be diagnosed, extracting total RNA thereof, preparing a first strand cDNA of the mRNA species in the total RNA extract or poly-A-selected fraction(s) thereof, which cDNA comprises a suitable tail; performing a PCR using a PLAG gene-specific primer and a tail-specific primer in order to amplify PLAG gene-specific cDNA's; separating the PCR products on a gel to obtain a pattern of bands; evaluating the presence of aberrant bands by comparison to wildtype bands, preferably originating from the same individual.

As an alternative amplification may be performed by means of the Nucleic Acid Sequence-Based Amplification (NASBA) technique [Compton, J. (1991) Nucleic acid sequence-based amplification. Nature 350,91-92] or variations thereof.

In another embodiment the method comprises the steps of taking a biopsy of the tumor to obtain cells to be diagnosed, isolating total protein therefrom, separating the total protein on a gel to obtain essentially individual bands, optionally transfering the bands to a Western blot, hybridising the bands thus obtained with antibodies directed against a part of the protein encoded by the remaining part of the PLAG gene and against a part of the protein encoded by the substitution part of the PLAG gene; visualising the antigen-antibody reactions and establishing the presence of aberrant bands by comparison with bands from wildtype proteins, preferably originating from the same individual.

In a further embodiment the method comprises taking a biopsy of the tumor to obtain cells to be diagnosed; isolating total DNA therefrom; digesting the DNA with one or more so-called "rare cutter" (typically "6- or more cutters") restriction enzymes; separating the digest thus prepared on a gel to obtain a separation pattern; optionally transfering the separation pattern to a Southern blot; hybridising the separation pattern in the gel or on the blot with a set of probes under hybridising conditions; visualising the hybridisations and establishing the presence of aberrant bands by comparison to wildtype bands, preferably originating from the same individual.

Changes in the expression level of the gene may be detected by measuring mRNA levels or protein levels by means of a suitable probe.

Diagnostic methods based on abnormal expression levels of the gene may comprise the steps of taking a sample of the tumor to obtain cells to be diagnosed; isolating mRNA therefrom; and establishing the presence and/or the (relative) quantity of mRNA transcribed from the PLAG gene of interest in comparison to a control. Establishing the presence or (relative) quantity of the mRNA may be achieved by amplifying at least part of the mRNA of the PLAG gene by means of RT-PCR or similar amplification techniques. In an alternative embodiment the expression level may be established by determination of the presence or the amount of the gene product (e.g. protein) by means of for example monoclonal antibodies.

The diagnostic methods of the invention may be used for diseases wherein cells having a non-physiological proliferative capacity are selected from the group consisting of benign tumors, such as the tumors pleomorphic adenomas of the salivary glands, lipoblastomas, uterine leiomyomas, and other benign tumors as well as various malignant tumors, including but not limited to sarcomas (e.g. rhabdomyosarcoma) and leukemias and lymphomas.

As already indicated above, it has been found that in certain malignant tumors the expression level of the PLAG genes is increased. Another aspect of the invention thus relates to the implementation of the identification of the PLAG genes in therapy. The invention for example provides anti-sense molecules or expression inhibitors of the PLAG gene for use in the treatment of diseases involving cells having a non-physiological proliferative capacity by modulating the expression of the gene.

The invention thus provides derivatives of the PLAG gene and/or its immediate environment for use in diagnosis and the preparation of therapeutical compositions, wherein the derivatives are selected from the group consisting of sense and anti-sense cDNA or fragments thereof, transcripts of the gene or fragments thereof, antisense RNA, triple helix inducing molecule or other types of "transcription clamps", fragments of the gene or its complementary strand, proteins encoded by the gene or fragments thereof, protein nucleic acids (PNA), antibodies directed to the gene, the cDNA, the transcript, the protein or the fragments thereof, as well as antibody fragments. Besides the use of direct derivatives of the genes and their surroundings (flanking sequences) in diagnosis and therapy, other molecules, like expression inhibitors or expression enhancers, may be used for therapeutic treatment according to the invention. An example of this type of molecules are ribozymes that destroy RNA molecules.

Bsides the above-described therapeutic and diagnostic methods, the principles of the invention may also be used for producing a transgenic animal model for testing pharmaceuticals for treatment of PLAG-related malignant or benign tumors. One of the examples describes the production of such an animal model.

It is to be understood that the principles of the present invention are described herein for illustration purposes only with reference to the PLAG1 gene mapping at chromosome 8q12 and the CTNNB1 gene on chromosome 3p21. Based on the information provided in this application the skilled person will be able to isolate and sequence corresponding genes of the gene family and apply the principles of this invention by using the gene and its sequence without departing from the scope of the general concept of this invention.

The present invention will thus be further elucidated by the following examples which are in no way intended to limit the scope thereof.

### EXAMPLES

### EXAMPLE 1

### 1. Introduction

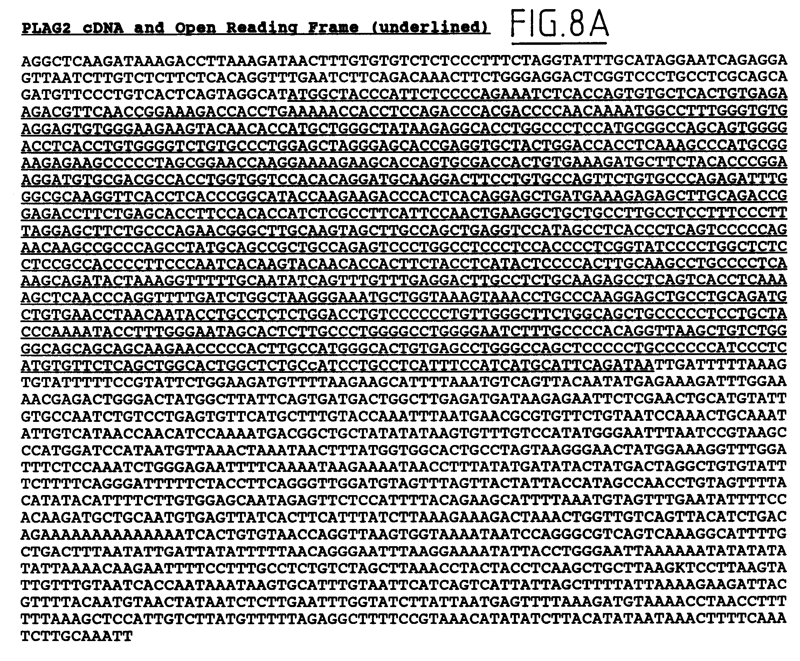

This example describes the isolation and analysis of overlapping YAC clones and the establishment of a YAC contig (set of overlapping clones), which spans genomic DNA around the MOS locus and includes the translocation breakpoints, t(3;8)(p21;q12), of pleomorphic salivary glands (Fig. 8). Pleomorphic adenomas are benign epithelial tumors originating from the major and minor salivary glands. Cytogenetic analysis has indicated that these tumors mainly display chromosome breakpoints in region q12 of chromosome 8. In previous studies we have shown that these breakpoints are located in the 9 cM interval between MOS / D8S285 and D8S260. Here we report directional chromosome walking studies starting from D8S260 and D8S285, resulting in, respectively, a rudimental YAC contig of 5 Mb and a highly detailed YAC contig and long-range physical map of about 2 Mb. The latter YAC contig has at least double coverage and consists of 34 overlapping YAC clones isolated from the CEPH and ICRF YAC libraries. Their insert sizes were estimated by contour-clamped homogeneous electric field (CHEF) gel electrophoresis. Chromosomal localization of the YACs was investigated by FISH analysis. On the basis of YAC insert and insert-end derived DNA markers and (publicly known) sequence tag sites (STSs), with an average spacing of 65 kbp, as well as restriction enzyme analysis, a long-range physical map was established for this centromeric region of chromosome 8q12. Within the YAC contig, the relative positions of various known genes and expressed sequence tag sites was determined. This YAC contig constitutes the basis for the construction of a transcriptional map of this region. FISH analysis using YACs and cosmids, corresponding to STSs in this YAC contig, already revealed that all but two of the chromosome 8q12 breakpoints in the primary tumors that were tested, mapped within a 300 kb interval between the MOS proto-oncogene and STS EM156.

### 2. Materials and methods

### 2.1. Pleomorphic adenomas of the salivary glands and cytogenetic analysis.

Primary pleomorphic adenomas of the salivary glands were obtained from patients at the time of surgery. Primary cultures and chromosome preparations were established and analyzed as described before (Röijer et al., 1996). The primary tumors with their aberrations used in this study are listed in Table 1. Slides for FISH were prepared from cells stored in fixative at -20°C.

### 2.2. Fluorescence in situ hybridization (FISH)

Slides for FISH were aged for 2-10 days, or were treated with 2x SSC (pH 7.0) at 37°C for 30 min, and subsequently denatured in 70% formamide in 2x SSC (pH 7.0), at 75°C for 2-6 minutes. Cosmid and YAC DNAs (1 µg) were labeled with biotin-16-dUTP (Boehringer Mannheim) by nick translation. Labeled DNA was purified through a Sephadex G50 column (Pharmacia), coprecipitated with 50 mg sonicated human placenta DNA (Sigma), and dissolved in hybridization solution (50% formamide, 2x SSC pH 7.0, 50 mM sodium phosphate pH 7.0, 10% dextran sulfate). Hybridization and probe detection were carried out as previously described (Röijer et al., 1996). The alpha-satellite probe D8Z2 was obtained from Oncor. Slides were examined in a Zeiss Axiophot epifluorescence microscope using the appropriate filter combinations. Fluorescence signals were digitalized, enhanced and analyzed using the ProbeMaster FISH image analysis system (Perceptive Scientific Instruments, Houston, Texas). Color prints were produced using a Kodak XL 7700 monochrome continuous printer.

### 2.3 DNA preparations.

Extraction of genomic DNA and the Southern analyses were performed as described previously (Schoenmakers et al., 1994). DNA from YACs, cosmids, PCR products and oligonucleotides was labelled using a variety of techniques. For FISH, cosmid clones or inter-Alu PCR products of YACs were biotinylated with biotin-11-dUTP (Boehringer) by nick translation. YAC DNA (100 ng) was amplified by inter Alu PCR (P1: CTGCACTCCAGCCTGGG, P2: TCCCAAAGTGCTGGGATTACAG). After initial denaturation for 5 min at 94°C, 30 amplification cycles were performed each consisting of denaturation for 1 min at 94°C, annealing for 30 sec at 37°C and extension for 6 min at 72°C, and with a final extension at 72°C for 10 min. Amplified DNA was purified with QIAQuick PCR Purification kit (Qiagen). For filter hybridizations, probes were radio-labelled with alpha-³²P-dCTP using random hexamers (Feinberg and Vogelstein, 1984). In case of PCR-products smaller than 200 bp in size, a similar protocol was applied, but specific oligonucleotides were used to prime labelling reactions. Oligonucleotides were labelled using gamma-³²P-ATP.

### 2.4 YAC library screening.

YAC clones in this paper were isolated from the CEPH mark 1 (Albertsen et al., 1990) and CEPH mark 3 (Chumakov et al., 1992) YAC library, made available to us by the Centre d'Etude du Polymorphisme Humain (CEPH). YACs were isolated using a combination of PCR-based screening and colony hybridization analysis (Green and Olson, 1990). Contaminating Candida parapsylosis, which was sometimes encountered, was eradicated by adding terbinafin to the growth medium (final concentration of 25 microgram/ml). The isolated YAC clones were characterized by STS-content mapping, contour-clamped homogeneous electric field (CHEF) electrophoresis (Chu et al., 1986), restriction mapping and hybridization and FISH analysis.

### 2.5 Cosmid library screening.

Cosmid clones were isolated from an arrayed human chromosome 8-specific cosmid library (Wood et al., 1992) obtained from Los Alamos National Laboratory (LANL). LANL-derived cosmid clones are indicated by their microtiter plate addresses. Cosmid DNA was extracted using standard techniques involving purification over Oiagen tips (Qiagen).

### 2.6 Pulsed-field gel electrophoresis and Southern blot analysis.

Pulsed-field gel electrophoresis and Southern blot analysis were performed exactly as described by Schoenmakers et al. (1994). Agarose plugs containing high-molecular weight YAC DNA (equivalent to about 1 x 108 yeast cells) were twice equilibrated in approximately 25 ml TE buffer (pH 8.0) for 30 min at 50 °C followed by two similar rounds of equilibration at room temperature. Plugs were subsequently transferred to round-bottom 2 ml eppendorf tubes and equilibrated two times for 30 min in 500 microliter of the appropriate 1 x restriction-buffer at the appropriate restriction temperature.

Thereafter, DNA was digested in the plugs according to the suppliers (Boehringer) instructions for 4 h using 30 units of restriction endonuclease per digestion reaction. After digestion, plugs along with appropriate molecular weight markers were loaded onto a 1% agarose / 0.25 x TBE gel, sealed with LMP-agarose and size fractionated on a CHEF apparatus (Biorad) for 18 h at 6.0 V/cm using a pulse angle of 120 degrees and constant pulse times varying from 10 sec (separation up to 300 kbp) to 20 sec (separation up to 500 kbp). In the case of large restriction fragments, additional runs were performed, aiming at the separation of fragments with sizes above 500 kbp.

Electrophoresis was performed at 14°C in 0.25 x TBE. As molecular weight markers, lambda ladders (Promega) and home-made plugs containing lambda DNA cut with restriction endonuclease HindIII were used. After electrophoresis, gels were stained with ethidium bromide, photographed, and UV irradiated using a stratalinker (Stratagene) set at 120 mJ. DNA was subsequently blotted onto Hybond N+ membranes (Amersham) for 4-16 h using 0.4 N NaOH as transfer buffer. After blotting, the membranes were dried for 15 min at 80°C, briefly neutralised in 2 x SSPE, and prehybridised for at least 3 h at 42°C in 50 ml of a solution consisting of 50% formamide, 5 x SSPE, 5 x Denhardts, 0.1% SDS and 200 microgram/ml heparin. Filters were subsequently hybridised for 16 h at 42°C in 10 ml of a solution consisting of 50% formamide, 5 x SSPE, 1 x Denhardts, 0.1% SDS, 100 microgram/ml heparin, 0.5% dextran sulphate and 2-3 x 106 cpm/ml of labelled probe. Thereafter, membranes were first washed two times for 5 min in 2 x SSPE/0.1% SDS at room temperature, then for 30 min in 2 x SSPE/0.1% SDS at 42°C and, finally, in 0.1 x SSPE/0.1% SDS for 20 min at 65°C. Kodak XAR-5 films were exposed at -80°C for 3-16 h, depending on probe performance. Intensifying screens (Kyokko special 500) were used.

Agarose plugs containing high-molecular weight yeast + YAC DNA (equivalent to 1 x 10⁹ cells ml⁻¹) were prepared as described before (Schoenmakers et al., 1994). Plugs were thoroughly dialysed against four changes of 25 ml T10E1 (pH 8.0) followed by two changes of 0.5 ml 1 x restriction buffer before they were subjected to either pulsed-field restriction enzyme mapping or YAC-end rescue.

### 2.7 PCR analysis.

PCR amplification was carried out using a Pharmacia LKB-Gene ATAQ Controller (Pharmacia/LKB) or a Perkin Elmer 9600 (Perkin-Elmer Cetus) in final volumes of respectively 50 and 25 microliter containing 10 mM Tris-HCl pH 8.3, 50 mM KCl, 1.5 mM MgCl₂, 0.01% gelatine, 2 mM dNTPs, 20 pmole of each amplimer, 1.25 units of AmpliTaq (Perkin-Elmer Cetus), and 100 ng (for superpools) or 20 ng (for pools) of DNA. After initial denaturation for 5 min at 94°C, 30 amplification cycles were performed each consisting of denaturation for 1 min at 94°C, annealing for 1 min at the appropriate temperature (see Table 3), and extension for 1 min at 72°C, and with a final extension at 72°C for 10 min. Results were evaluated by analysis of 10 microliter of the reaction product on 2% agarose gels.

### 2.8 Generation of STSs from YAC inserts and insert ends.

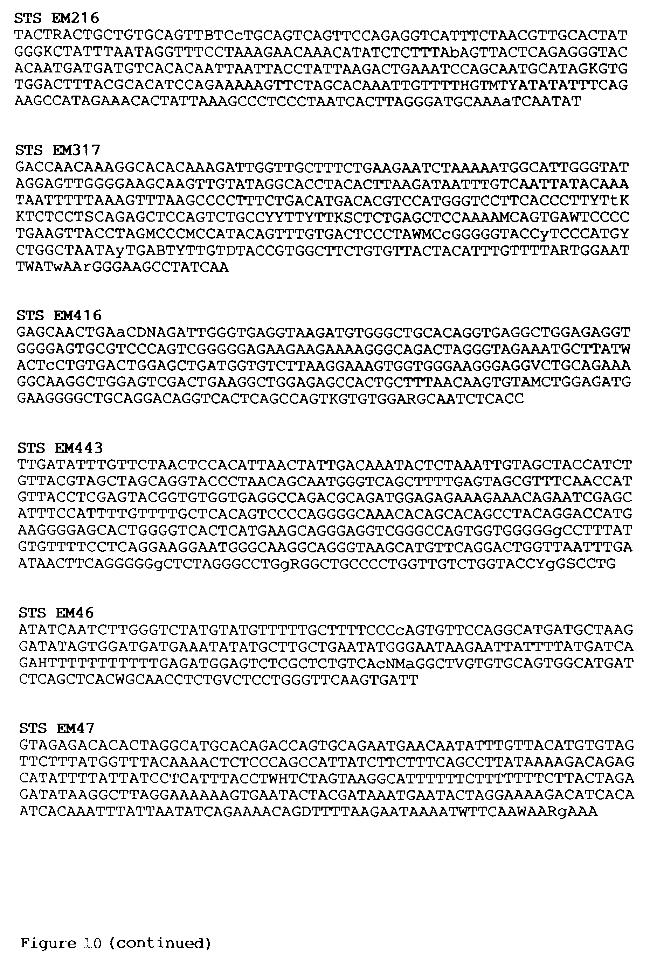

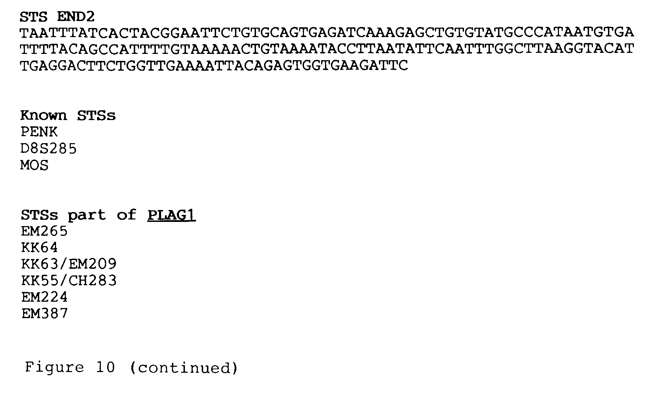

YAC-end rescue was performed using a vectorette-PCR procedure in combination with direct solid phase DNA sequencing, as described before (Geurts et al., 1994). STSs specific for YAC inserts were generated from inter-Alu PCR products, isolated using published oligonucleotides TC65 or 517 (Nelson et al., 1989) to which SalI-tails were added to facilitate cloning. Figure 10 shows some of these STSs. After sequence analysis, primer pairs were developed using the OLIGO computer algorithm (Rychlik, 1989). They were tested on human genomic DNA, basically according to procedures described above.

### 2.9 Nucleotide sequence analysis and oligonucleotides.

Nucleotide sequences were determined according to the dideoxy chain termination method using the T7 polymerase sequencing kit of Pharmacia/LKB or the dsDNA Cycle Sequencing System (GIBCO/BRL). Sequencing results were analyzed using an A.L.F. DNA sequencer™ (Pharmacia Biotech) on standard 30 cm, 6% Hydrolink®, Long Range™ gels (AT Biochem). Sequence analysis utilized Lasergene (DNASTAR) and BLAST and BEAUTY searches (NCBI; Altschul et al., 1990). All oligonucleotides were purchased from Pharmacia Biotech.

### 3. Results

### 3.1 Isolation of YAC clones to start chromosome walking.

According to radiation hybrid mapping data, band 8q12 is flanked by markers D8S165, D8S285 proximally, and by D8S260 distally (Sapru et al., 1994). The estimated genetic distance between these markers is 9 cM (Gyapay et al., 1994). Using primer sets for these microsatellite markers, we selected sets of corresponding YAC clones. These YACs were used for initiating the walk towards the 8q12 translocation breakpoints, meanwhile generating a long-range contig based upon STS-content mapping of these. Hence we started to construct a 8q12 centromeric YAC contig (from D8S165 / D8S285) and a 8q12 telomeric YAC contig (from D8S260). By fluorescent in situ hybridisation (FISH) experiments, we could soon show that all but two of the translocation breakpoints in primary adenomas mapped within a 1 cM interval within the centromeric YAC contig (Röijer et al., 1996). Hence, the YAC contig of the telomeric side of 8q12 was not characterized in that much detail. The two YAC contigs could not be linked to each other.

### 3.2 Assembly of a YAC contig starting from D8S285

In previous studies (Röijer et al., 1996) we have described the isolation of two overlapping YACs containing D8S285, namely 935E9 and 166F4. These YACs, together with a MOS containing YAC (900g10157), were used as a starting point for a chromosome walking project to define the 8q12 centromeric region, as mentioned in the introduction. Initially, chromosome walking was performed bidirectionally. This allowed us to hook up our 8q12 centromeric contig to a chromosome 8q11 YAC contig. Both contigs have the marker D8S593 in common. In the subsequent PCR based screening of the human CEPH YAC libraries with D8S108 and D8S166, we isolated MegaYAC 946B7, which together with MegaYAC 935E9, formed the backbone of our centromeric contig.

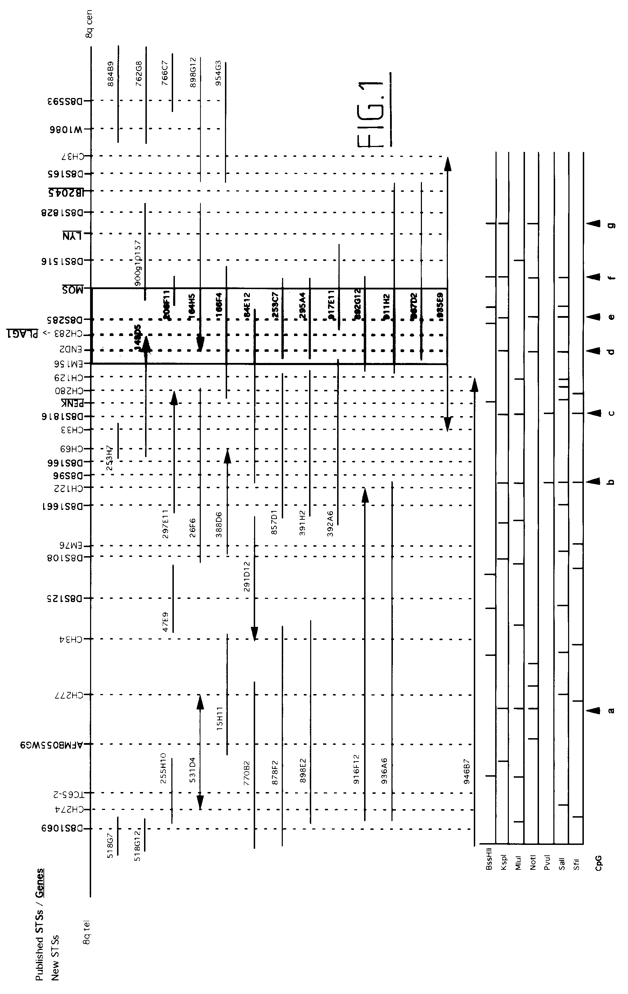

In the bidirectional and subsequent unidirectional chromosome walking steps, the following general procedures were used. First, rescuing and sequencing the ends of YAC clones resulted in DNA markers characterizing their left and right sides (Table 2). Based on sequence data on the ends of the YAC insert, as well as on inter-Alu PCR products, primer sets were developed for specific amplification of DNA, establishing STSs (Table 3). Their location to 8q12-qter was determined by CASH as well as by FISH after corresponding YAC and/or cosmid clones were isolated. With the sequentially selected and evaluated primer sets, screening of the YAC and cosmid libraries was performed to isolate the building blocks for contig assembly. Hence, we established a 8q12 centromeric YAC contig consisting of 34 overlapping YAC clones, covering approximately 2 Mb of DNA (Fig. 1). The 34 YACs are between 160 kb and 1660 kb long. This YAC contig appeared to encompass the chromosome 8q12 breakpoints of all but two primary adenomas studied. YAC 166F4 for instance, was already shown to cross the translocation breakpoint in two adenomas with t(3;8)(p21;q12) (Röijer et al., 1996). Characteristics of the YACs that were used to build this contig are given in Table 2.

The YAC contig was constructed in parallel with the screening data of Whitehead Institute / MIT Center for Genome Research available via http://www-genome.wi.mit.edu/cgi-bin/contig/lookup_contig (Contigs WC8.7 and WC-157).

### 3.3 Physical mapping of polymorphic markers, ESTs and genes

All markers and genes which were or became publicly available during our work were STS content mapped on our contig and those found positive were subsequently sublocalized by (primer) hybridization on YAC Southern blots. The markers that were found to reside within the centromeric contig presented here were D8S1069 (WI-536), AFMB055WG9, D8S125, D8S108, D8S1661 (WI-5459), D8S166, D8S96, D8S1816, D8S285, D8S1516 (WI-3862), D8S1828 and D8S165. Furthermore, besides the MOS proto-oncogene (Testa et al., 1988), two other genes could be positioned in our centromeric YAC contig, namely the YES related proto-oncogene LYN (Corey and Shapiro, 1994) and the preproenkephalin gene PENK (Tomfohrde et al., 1992). Finally, our contig contains 2 expressed sequence tags, namely WI-7754 (D8S1930), corresponding to LYN, and IB2045, not resembling any currently known gene. The genes encoding the kappa opioid receptor (OPRK1), (Yasuda et al., 1994) and interleukin-7 (IL7) (Brunton and Lupton, 1990) are not contained in our YAC contig.

### 3.4 Restriction map and putative CpG islands: Construction of a rare-cutter physical map from the 2 Mb YAC contig in the 8q12 centromeric region

Southern blots of total yeast plus YAC DNA, digested to completion with rare-cutter enzymes BssHII, KpnI, MluI, NotI, PvuI, SalI and SfiI (see Materials and Methods) and separated on CHEF gels, were hybridized sequentially with (i) the STS used for the initial screening of the YAC in question, (ii) pYAC4 right arm sequences, (iii) pYAC4 left arm sequences, and (iv) a human Alu-repeat probe (BLUR-8). The long-range restriction map that was obtained in this way was completed by probing with PCR-isolated STSs/YAC end probes. Restriction maps of individual YAC clones were aligned, and a consensus restriction map was established. The region was searched for CpG islands on the basis of the colocalization of sites for these rare-cutter enzymes (Fig. 1).

### 3.5 FISH mapping of 8q12 breakpoints in pleomorphic adenomas

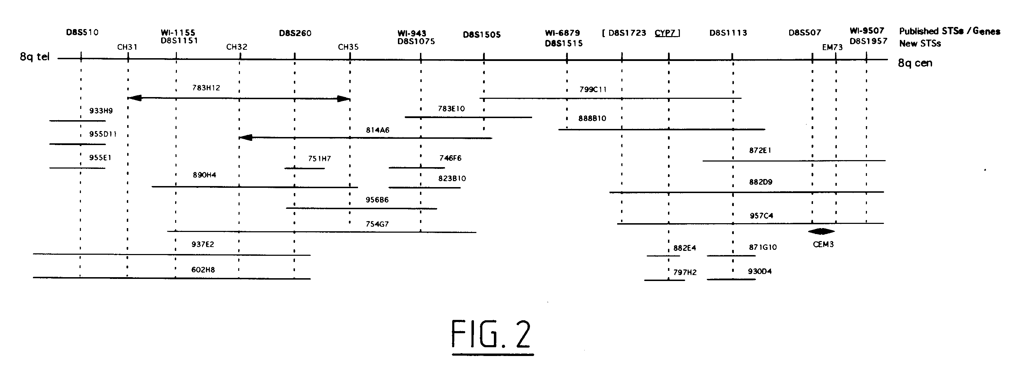

In previous studies we have mapped the t(3;8)(p21;q12) breakpoint within a 1 Mb region flanked by MOS proximally and by the genetic marker D8S166 distally. One YAC within this region (166F4) was shown to span the t(3;8) breakpoint in two primary pleomorphic adenomas (Röijer et al. (1996)). We have now extended these previous observations by analysis of additional tumors including cases with different variant translocations (Table 1). Six of eight primary adenomas with 8q12 abnormalities had breakpoints mapping within YAC 166F4 (Fig. 2A). Also rumors with other 8q12 rearrangements than the classical t(3;8) had breakpoints within the same YAC, demonstrating that this is the major breakpoint cluster region in pleomorphic adenomas with 8q12 abnormalities.

To fine map this region we isolated cosmids from an arrayed human chromosome 8-specific cosmid library (obtained from Los Alamos National Laboratory; Wood et al. (1992)) using markers contained within YAC 166F4. Cosmid clones containing MOS, EM156 and CH129 were isolated and mapped by FISH (Fig. 1). The breakpoints in all six cases were localized between cosmids CEM1 (MOS) en CEM23 (EM156) (Fig. 2B and C). In for example CG 666, CEM1 was transposed to 8p due to a pericentric inversion of the dicentris marker chromosome (Fig. 2B), while CEM23 remained in its normal position at 8q12 (Fig. 2C). In adenoma CG 682, which has an ins(8;3)(q12;p21.3p14.1), CEM23 spanned the insertion breakpoint (Röijer et al. (1997)).

Two of the eight adenomas tested, CG 650 and CG 787, had breakpoints clearly proximal to MOS. To further map these breakpoints we isolated two additional sets of YACs corresponding to CH37, D8S593 and the recently identified XRCC7 gene (Blunt et al. (1995)), respectively. In CG 650 the breakpoint was found to reside between ICRF YAC 900g10157 and YAC 898G12 (Fig. 1), and in CG 787 the breakpoint was found to reside within the XRCC7 containing YAC 943C4 (Fig. 2D). In the latter case, which judged from cytogenetics had an ins(8;7)(q11-12;p21p14), FISH analysis revealed signals on both the der(7) and the der(8) chromosomes, demonstrating that this is not a simple 8:7-insertion but a complex rearrangement with at least two breakpoints on 8q, one of which maps within YAC 943G4 and the other proximal to this YAC.

### 3.6 Assembly of a YAC contig between D8S260 and D8S507

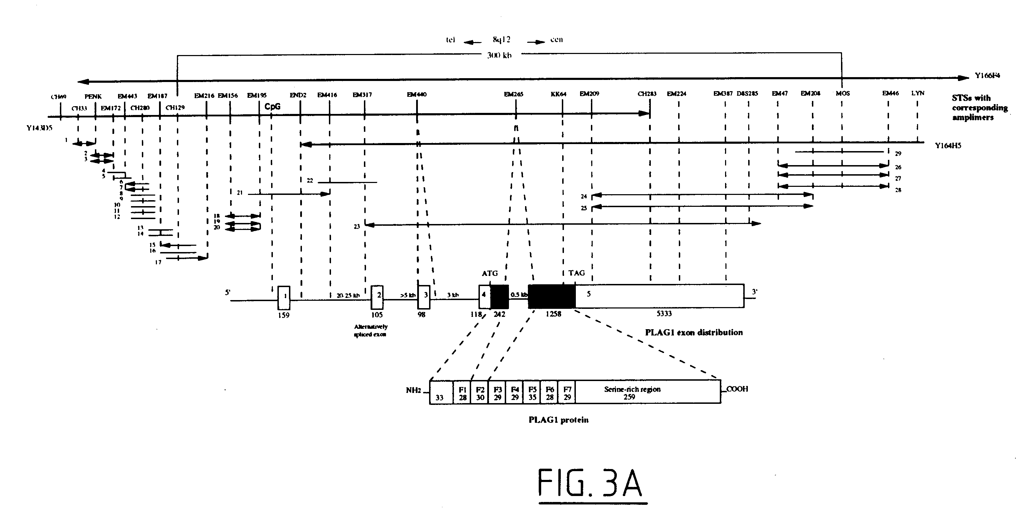

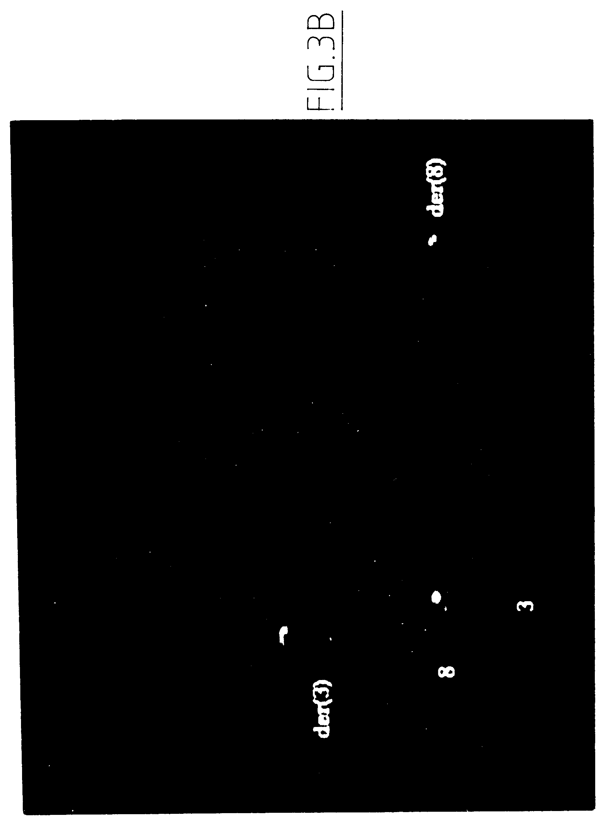

Two MegaYACs corresponding to D8S260, were used to start the chromosome walk from the telomeric side of chromosome 8q12.The YAC insert-ends from these two clones (783H12 and 814A6) were isolated and used for STS content mapping. The most telomeric STS (CH31), is present in two YACs also containing D8S510, and hence links the 8q12 YAC contig to a 8q13 specific YAC contig (Koenig et al., 1995). When we started our physical mapping effort, only one polymorphic marker, D8S507, was described in between D8S285 and D8S260 (Gyapay et al., 1994). Initially, we were unable to isolate YACs containing D8S507. In stead we used the corresponding amplimers to isolate a cosmid containing D8S507 (cosmid CEM3). An insert-ends of this cosmid clone (EM73) was then used to isolate corresponding MegaYACs (872E1, 882D9 and 957C4). It is unclear why these YACs were initially not recognised upon the YAC library screenings. This might have been due to either a less efficient PCR of D8S507 than of EM73, or to an underrepresentation of the D8S507 YAC-containing yeast cells in the primary pools of the specific library we screened. The availability of two new polymorphic markers, D8S1505 and D8S1515 (WI-6879) allowed to link the two separate MegaYAC contigs around D8S260 and D8S507. The composite contig consists of 23 CEPH MegaYACs and covers approximately 5 Mb of genomic DNA (Fig. 3). The polymorphic markers that were tested and found to reside in the telomeric YAC contig are D8S1151 (WI-1155), D8S260, D8S1075 (WI-943), D8S1505, D8S1515 (WI-6879), D8S1723, D8S507 and D8S1957 (WI-9507).

One gene, CYP7, encoding cholesterol 7a-Hydroxylase (and previously assigned to 8q11-12 using both mouse-human somatic cell hybrids and FISH (Cohen et al., 1992)) was mapped in this contig using a primerset that we developed on publicly available sequence data (see Table 3). The YAC contig was constructed in parallel with the screening data of Whitehead Institute / MIT Center for Genome Research available via http://www-genome.wi.mit.edu/cgi-bin/contig/lookup_contig (Contigs WC8.8 and WC-727).

### 4. Discussion

In this example the physical map of 2 Mb of the centromeric region of chromosome 8q12 composed of 34 YAC clones is presented. It is tagged by 31 markers with an average interval of 65 kb and was validated by FISH mapping. The frequency of chimaerism in the set of YACs studied in detail (YAC end rescue and/or restriction enzyme mapping and/or FISH) was slightly lower (6/26 = 23%) than the 30-40% expected (Van Ommen, 1993). These data provide the most detailed available physical map of the 8q12 region. The map was constructed in parallel with the establishment of a low-resolution map of the human genome and with the screening data of the Whitehead Institute/MIT Center for Genome Research. It was compared with data available electronically via Internet. The order of the markers on the physical map is in agreement with the 1996 Genethon human genetic linkage map (Dib et al. (1996)). In total, 31 markers/genes were ordered on the contig as follows: D8S1069-CH274-TC65.2-AFMB055WG9-CH277-CH34-D8S125-D8S108-EM76-D8S1661-CH122-D8S96-D8S166-CH69-CH33-D8S1816-PE-NK-CH280-CH129-EM156-END2-CH283-D8S285-MOS-D8S1516-LYN-D8S1-828-IB2045-D8S165-CH37-W1086.

A restriction map with the enzymes BssHII, KpnI, MluI, NotI, SalI, SfiI, SwaI was generated, revealing at least 7 putative CpG islands. Three of these island correspond to previously known genes (PENK, MOS and LYN), while the others might represent the 5' end of unidentified genes so far. Our 2 Mb map integrates cytogenetic, genetic and physical data of chromosome band 8q12. Chromosome band 8q12 seems to be very gene-poor: so far only 4 genes were shown to reside in both contigs (CYP7, PENK, MOS and LYN). This is consistent with the view that Giemsa-positive bands are chromosome regions rather low in G+C content and poor in genes (Craig and Bickmore, 1993). Chromosome 8q12 is partially conserved in synteny with mouse chromosome 4 (Mock et al., 1996). Comparative gene mapping however did not reveal any mouse gene or marker not present on our physical map.

Our physical map contains at least one disease locus, i.e. the pleomorphic adenoma translocation breakpoint. FISH analyses using YACs and cosmids revealed that the majority of breakpoints clustered within a 300 kb region in both adenomas with the recurrent t(3;8) and in those with different sporadic 8q12 translocations. A cosmid from this region was also shown to span the breakpoint in an adenoma with an ins(8;3) (Röijer et al. (1997)). This region is therefore likely to harbor the pleomorphic adenoma gene. The breakpoint region contains one known gene (MOS), one polymorphic marker (D8S285), and three new STSs (EM156, END2 and CH283). BLAST searches of these STSs revealed that CH283 displayed sequence identity with a publicly available EST (expressed sequence tag).

Two of the eight tumors in the present series had breakpoints outside the 300 kb breakpoint region. By FISH we could demonstrate that the breakpoints in both cases were clearly proximal to MOS. In one case the breakpoint was mapped between MOS and YAC 898G12, and in the other case it was found to reside within YAC 943G4. The latter YAC maps at least 2 Mb centromeric to MOS. Since the two breakpoints are clearly different and also separate from those located in the 300 kb breakpoint region it is unlikely that they affect the same gene. However, we can of course not rule out the possibility that these cases have additional rearrangements that may have escaped detection with the DNA probes used, and that they in fact all have rearrangements of a common gene in the breakpoint region.

Recently, we have obtained molecular evidence for an additional breakpoint region distal to our telomeric contig (Röijer et al. (in manuscript)). Based on FISH using YACs derived from 8q13-21 we have estimated that these breakpoints are about 15 to 20 cM telomeric to the 8q12 breakpoint cluster region. Cytogenetically, these breakpoints are very difficult, or impossible to distinguish from the classical 8q12 breakpoints. This is also true for the breakpoints located centromeric to MOS. Collectively, our findings thus suggest that there are several genes in the proximal part of 8q that are affected by chromosome rearrangements in pleomorphic adenomas.

In addition to pleomorphic adenomas there are also a few other types of solid tumor with structural rearrangements involving 8q11-13, namely lipoblastomas (Dal Cin et al. (1994); Sawyer et al. (1994)), rhabdomyosarcomas (Mitelman (1994)) and renal cell carcinomas (Elfving (1996)). It should also be mentioned that pleomorphic adenomas of the lacrimal glands show recurrent rearrangements of 8q12 (Hrynchak et al. (1994)), including a t(3;8)(p21;q12). Because of the well known histopathological and clinical similarities between pleomorphic adenomas originating from the salivary and lacrimal glands it is likely that the 8q12 abnormalities in these tumor types affect the same gene. As for the other tumor types mentioned it remains to be shown whether the rearrangements in these cases also affect this gene. Preliminary FISH analysis of lipoblastoma with an 8q12 translocation breakpoint have shown that the breakpoint, as in pleomorphic adenomas, is distal to MOS (unpublished observations). Unfortunately no markers telomeric to MOS could be tested. FISH studies are now in progress to resolve these critical questions.

Finally, a second YAC contig was established between D8S260 and D8S507, consisting of 23 CEPH MegaYACs and covering approximately 5 Mb. Both contigs represent about 75% of human chromosomal band 8q12, which extends over approximately 9 Mb of DNA. They will provide molecular access to new, previously unidentified genes from this region and especially to those directly affected by the chromosome 8q12 aberrations in pleomorphic adenomas of the salivary gland.

### 6. Figure legends

### Figure 1.

Physical contig and STS marker map in proximal human 8q12. The upper line indicates the chromosome, with the telomere to the left and the centromere to the right. DNA markers and genes are ordered across the top of the figure. Genes are underlined and previously known markers are indicated in bold. YAC clones are represented as lines together with their names and are described in Table 2. STS locations are indicated by dashed lines. The length of the solid line indicates its approximative size. The size of YAC 391H2 and 392A6 is too large to fit within the constraints of the map, suggesting chimaerism. In these cases, the lenght of the YAC corresponds only to the markers it contains. YAC end clones used as STS are represented by arrows. Below the contig the consensus restriction maps for BssHII (B), KpnI (K), MluI (M), NotI (N), PvuI (P), SalI (S) and SfiI (Sf) are indicated. Verical arrows indicate putative CpG islands, defined as the colocalization of sites for (a) K, M, N; (b) K, M, P, S, Sf; (c) K, M, P, Sf; (d) K, N, S; (e) K, N, S; (f) B, N, S; (g) B, K, N, Sf.
The pleomorphic adenoma gene region is indicated by a gray shaded box.

### Figure 2.

Physical contig and STS marker map in distal human 8q12. The upper line indicates the chromosome, with the telomere to the left and the centromere to the right. DNA markers and genes are ordered across the top of the figure. Genes are underlined and previously known markers are indicated in bold. YAC clones and cosmid CEM3 are represented as lines together with their names. YAC end clones used as STS are represented by arrows.

### Figure 10.

STSs used to generate the 300 kb cosmid contig mapping at chromosome 8q12 and encompassing PLAG1.

### 7. Tables

**TABLE 1**

| **Pleomorphic adenomas with chromosome 8q12 rearrangements.** | | |
|---|---|---|
| **Case No.** | **chromosome 8q12 rearrangements** | **Other abnormalities present** |
| CG 580 | t(8;15)(q12;q14) | No |
| CG 588 | t(3;8)(p21;q12) | No |
| CG 644 | t(3;8)(p21;q12) | No |
| CG 650 | t(5;8)(p13;q12) | Yes |
| CG 666 | der(8;11)t(8;11)(p23;q13) inv(8)(p22q12) | Yes |
| CG 682 | der(3)del(3)(p23-24)del(3)(p21.3p14.1), der(8)dir ins(8;3)(q12;p21.3p14.1) | No |
| CG 714 | ins(8;7)(q11-12;p21p14)* | Yes |
| CG 787 | der(8)del(8)(p11)t(8;13)(q12;q13) del(13)(q14), der(13)t(8;13)(q12;q13) | Yes |

| | | |
|---|---|---|
| * the interpretation of the karyotype based on FISH is as follows: der(7)ins(7;8)(p22;q11.1q11.2) (wcp 7+, wcp 8-, Y943G4+), der(8)ins(8;7)(q11.2;p21p14)(wcp7+, wcp8+, Y943G4+) | | |

**TABLE 2**

| **Analysis of YAC clones in the centromeric contig** | | | | |
|---|---|---|---|---|
| CEPH code | Size(kb) | Landm. left Acc. No. | Landm. right Acc. No. | Chimeric |
| 518G7 | | CH131 | | Y (left) |
| 518G12 | | CH132 | | ND |
| 770B2 | 1410 | CH281 | CH286 | ND |
| 878F2 | 800 | CH282 | CH287 | ND |
| 255H10 | 390 | CH273 | CH272 | ND |
| 531D4 | 430 | CH274 | CH277 | ND |
| 15H11 | 430 | | | ND |
| 291D12 | 410 | CH29 | CH34 | Y (left) |
| 47E9 | 370 | | | ND |
| 26F6 | 460 | | | ND |
| 916F12 | 1230 | CH122 | CH128 | ND |
| 388D6 | 430 | CH69 | CH100 | ND |
| 297E11 | 350 | CH280 | CH285 | ND |
| 253H7 | 160 | CH279 | CH284 | ND |
| 143D5 | 260 | CH278 | CH283 | ND |
| 391H2 | 620 | | | Y |
| 392A6 | 600 | | | Y |
| 84E12 | 500 | CH289 | CH290 | ND |
| 166F4 | 700 | CH67 | CH73 | ND |
| 164H5 | 375 | END2 | END6 | ND |
| 206F11 | | END3 | END7 | Y (right) |
| 253C7 | 620 | END4 | END8 | ND |
| 295A4 | | CH68 | CH74 | Y (left) |
| 917E11 | 1380 | END1 | END5 | ND |
| 946B7 | 1370 | CH123 | CH129 | ND |
| 935E9 | 1660 | CH33 | CH37 | ND |
| Note. YAC clones were isolated from CEPH YAC libraries as described under Materials and Methods. ND, not detected by methods used (FISH, YAC end rescue, restriction enzyme mapping). GenBank accession numbers are given. | | | | |

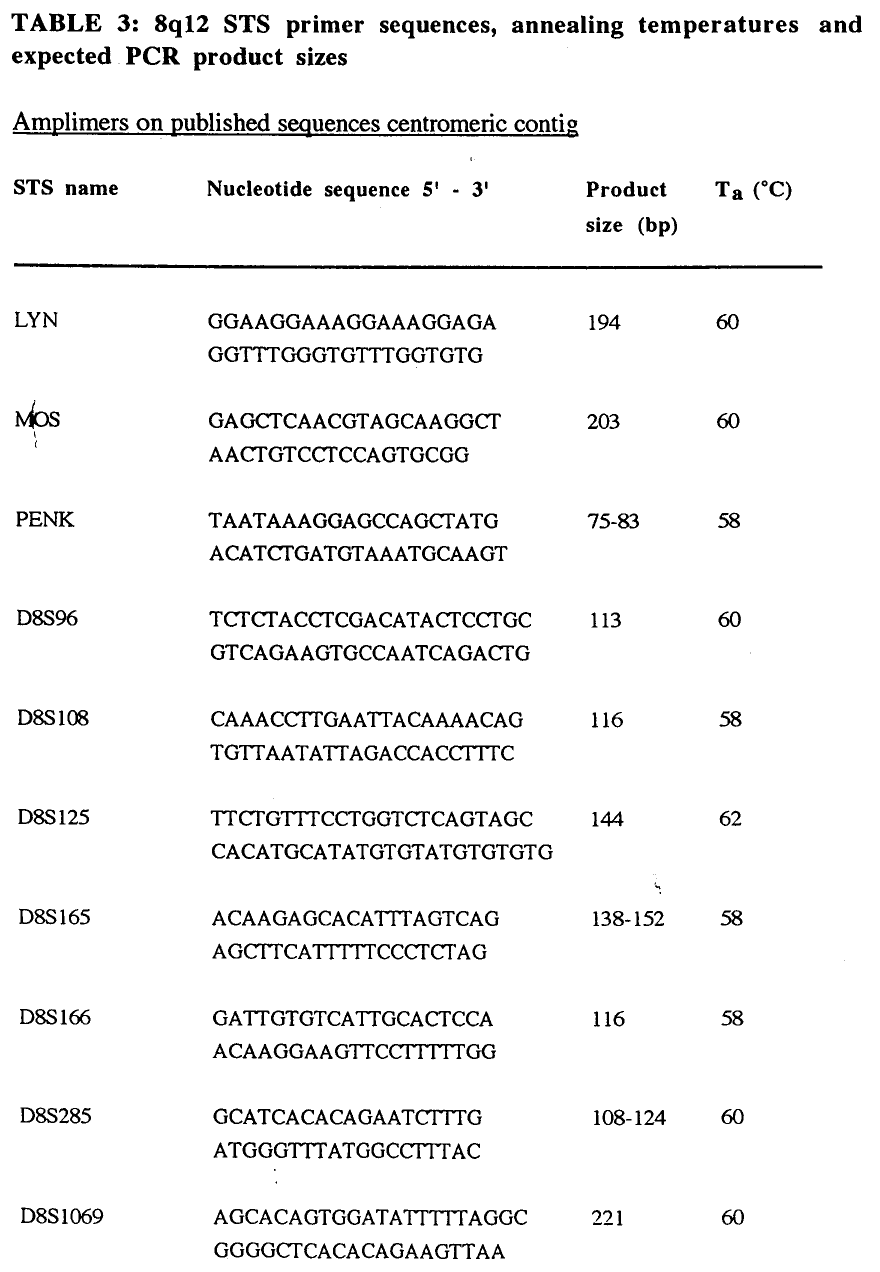

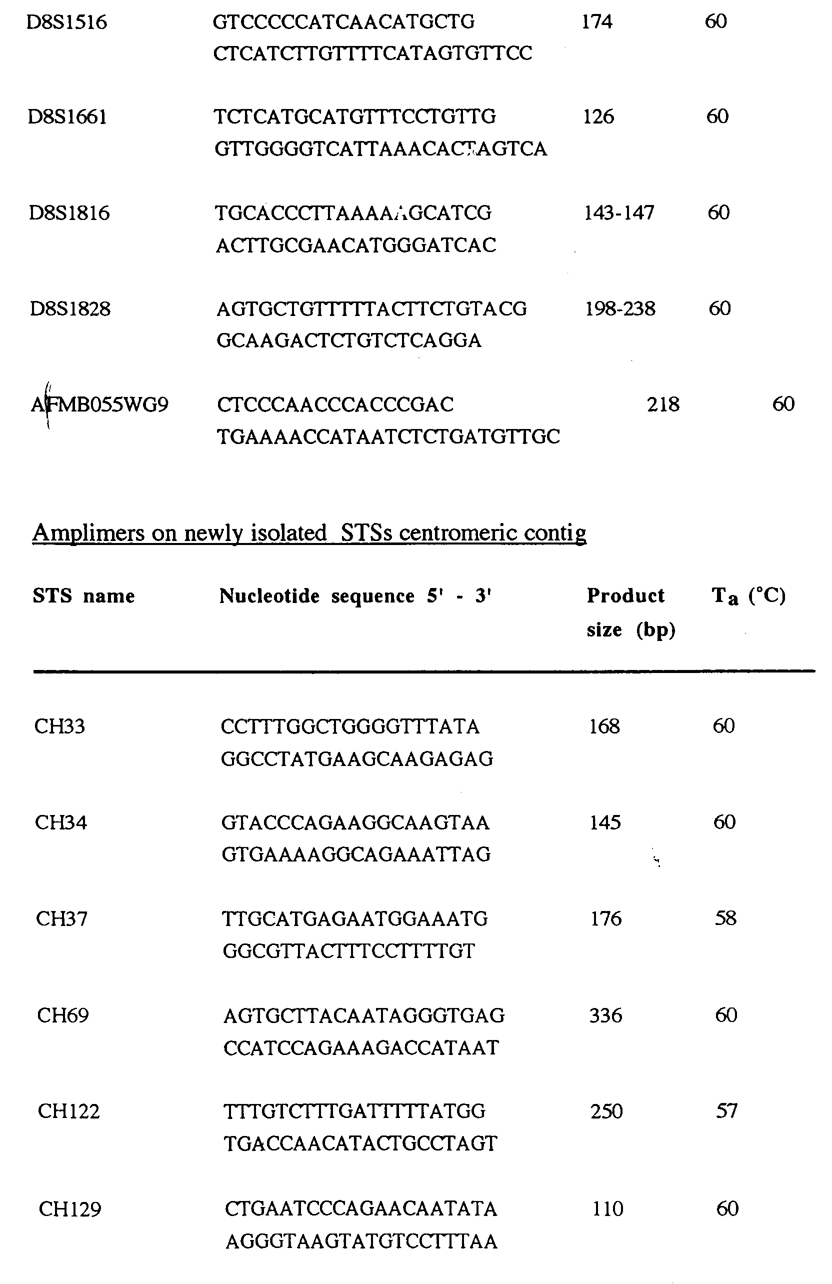

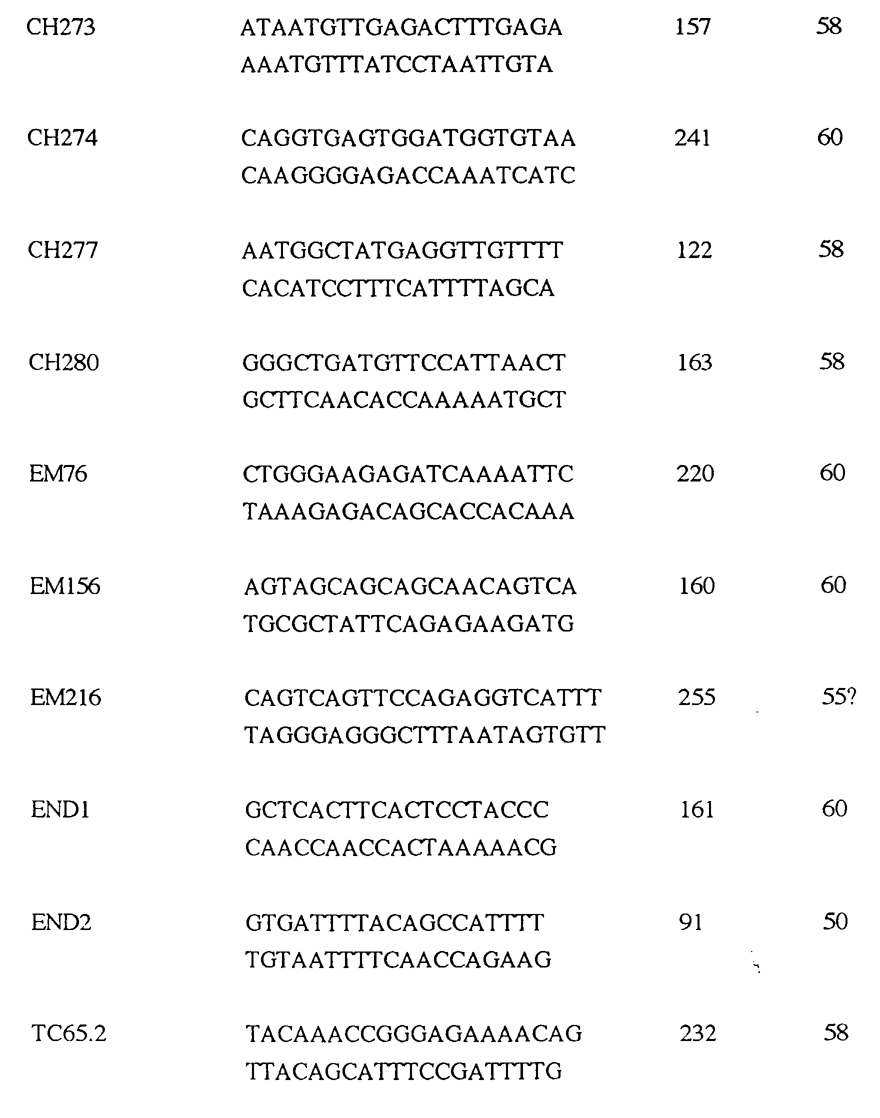

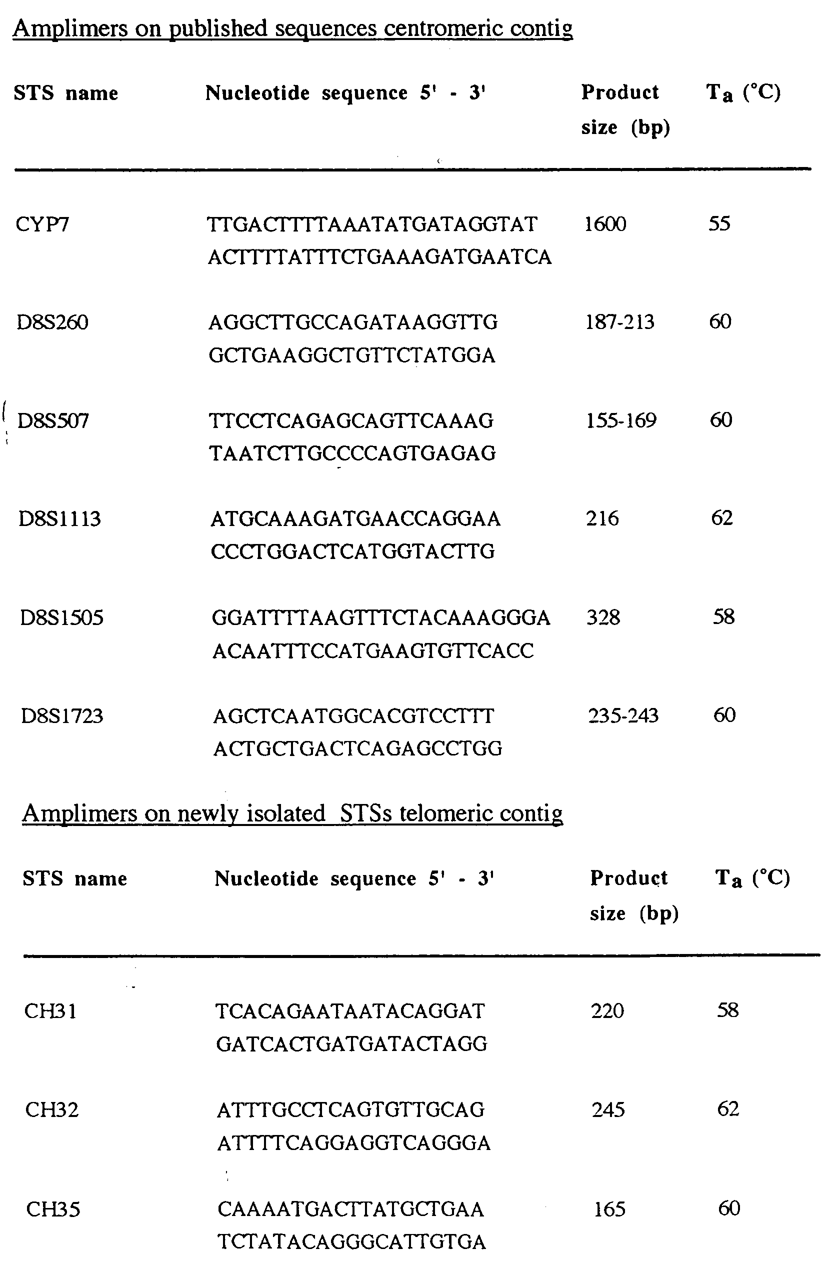

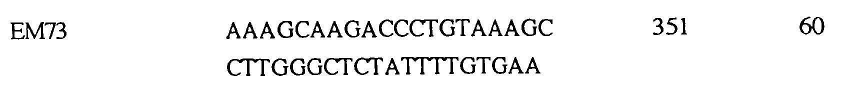

### EXAMPLE 2

### 1. Introduction

In this example the identification of a member of the PLAG gene family that appears to be of pathogenetical relevance is described. In previous experiments, it was found that the segment between MOS and PENK on human chromosome 8q12 harbors the recurrent chromosome t(3;8) (p21;q12) breakpoint frequently found in pleomorphic adenomas of the salivary glands. Using a positional cloning approach, the PLAG1 gene was identified and its genomic organization characterized.

The largest cytogenetic subgroup of pleomorphic adenoma of the salivary glands carries chromosome 8q12 aberrations with 3p21 as preferential translocation partner. Here we demonstrate that the t(3;8)(p21;q12) results in promoter swapping between PLAG1, a novel, developmentally regulated zinc finger gene on 8q12, and the constitutively expressed gene for β-catenin (CTNNB1), a protein interface functioning in adherens junctions and the WG/WNT signalling pathway. Fusions occur in the 5'-non-coding parts of both genes, exchanging regulatory control elements while preserving the coding regions. Due to the t(3;8)(p21;q12), PLAG1 is activated and expression of CTNNB1 down-regulated. Activation of PLAG1 was also observed in an adenoma with a variant translocation t(8;15). The results indicate that PLAG1 activation due to promoter swapping is a crucial event in salivary gland tumorigenesis.

Recently, molecular insight was obtained into the genetic basis of benign tumors by the discovery of a common genetic denominator in tumors of different types but with a particular cytogenetic profile (Schoenmakers et al. (1995); Ashar et al. (1995)). Molecular analysis of recurrent chromosome 12q13-15 aberrations in pleomorphic adenoma of the salivary glands, lipoma, uterine leiomyoma, hamartoma of lung and breast, fibroadenoma of the breast, angiomyxoma, and endometrial polyps revealed the consistent involvement of the high mobility group protein gene HMGIC; the first "benign oncogene".

Evidence is mounting that the same is likely to hold true for the closely related family member HMGIY, which is located on the short arm of chromosome 6. Both genes belong to the high mobility group (HMG) protein gene family (Bustin et al. (1990). Their corresponding proteins possess three amino-terminal AT hooks, through which the proteins are assumed to bind to A/T-rich DNA sequences, and an acidic tail in the carboxy-terminal region. Functionally, they act as architectural factors in the nuclear scaffold, critical for the correct assembly of stereospecific transcriptional complexes (Wolffe (1994)).

The genetic aberrations frequently result in the separation of the three AT hooks from the acidic tail, although all the pivotal changes in the high mobility group protein genes that can lead to aberrant growth control are not yet known. It should be noted that a variety of chromosome segments can act as translocation partner of 12q13-15 but each tumor type seems to have a preferential translocation partner. At present, only the preferential t(3;12)(q27-28;q15) translocation of ordinary lipoma has been characterized (Petit et al. (1996)). The gene affected on chromosome 3 is the LPP gene, which encodes a protein containing three LIM domains. LIM domains have been found in a growing number of proteins in mammals, amphibians, flies, worms, and plants and act as modular protein-binding interfaces (Schmeichel & Beckerle (1994)). LIM proteins mainly have a function in cell signalling and developmental regulation and include, for instance, transcription regulators, proto-oncogene products, and adhesion plaque constituents. The preferential t(3;12) in lipoma results in the formation of an HMGIC/LPP fusion transcript encoding a hybrid protein consisting of the three DNA binding domains of HMGI-C and the LIM domains of the protein encoded by LPP.

In an approach to systematically unravel the molecular pathogenesis of benign tumors, the inventors have focused on other cytogenetic subgroups of these, since their mere existence indicate the presence of additional critical genes. It is of interest to establish whether such genes are functionally similar or different as compared to the high mobility group protein genes HMGIC and HMGIY. In this context, the inventors have now molecularly characterized the largest cytogenetic subgroup of pleomorphic adenomas. This type of neoplasm is an epithelial tumor occurring primarily in the major and minor salivary glands. It is by far the most common type of salivary gland tumor, accounting for almost 50 % of all tumors in these organs (Seifert et al. (1990)). Pleomorphic adenomas are almost exclusively benign tumors, which only rarely undergo malignant transformation. They show a marked histological diversity with epithelial and myoepithelial cells arranged in a variety of patterns in a matrix of mucoid, myxoid, chondroid and, on rare occasions, even osteoid tissues. Cytogenetically, pleomorphic adenomas are characterized by recurrent rearrangements, in particular reciprocal translocations with consistent breakpoints at 3p21, 8q12 and 12q13-15 (Mitelman (1994)). In addition to the cases with abnormal karyotypes, there is also a subgroup of tumors with apparently normal karyotypes (30 to 50% of the cases). Abnormalities of 8q12 are most common and are found in about 60% of the cases with abnormal stemlines, whereas rearrangements of 3p21 and 12q13-15 are found in about 30% and 20% of the cases, respectively. The most frequent and characteristic abnormality so far observed in pleomorphic adenomas is a reciprocal t(3;8)(p21;q12) translocation (Mark et al. (1980)). While chromosome 3p is the preferential translocation partner, most human chromosomes have been found as translocation partner of 8q12. Rearrangements of 8q12 are often found as the sole anomalies, indicating that they represent primary cytogenetic events of possible pathogenetic importance.

Here, the inventors describe the positional cloning of the 8q12 translocation breakpoint as well as the identification and characterization of the genes on chromosome 8q12 and 3p21 disrupted by the t(3;8) translocation. The gene on chromosome 8q12 is a novel zinc finger gene, which we have designated PLAG1, whereas the gene on 3p21 is CTNNB1 which is the translocation partner, the gene for β-catenin, a protein with an established role in cell adhesion and signal transduction (Peifer (1993)). By Southern blot and RACE analysis, we pinpoint the breakpoints within the introns of the 5'-noncoding regions of both genes and show that well-defined fusion transcripts are expressed in the tumor cells. We also demonstrate that the t(3;8) translocation results in specific disruption of the transcriptional control of the two genes, leading to activation of PLAG1 and down-regulation of CTNNB1. A pleomorphic adenoma with 8q12 but not 3p21 involvement has also been studied to evaluate the phenomenon of disruption of transcriptional control in a broader perspective.

### 2. Materials and methods

### 2.1 Tumor material and chromosome analysis

Primary pleomorphic adenomas of the salivary glands were obtained from patients at the time of surgery. Primary cultures and chromosome preparations were made and analyzed as described by Röijer et al. (1996). Primary pleomorphic adenomas, including CG368, CG580, CG644, CG752, CG753 and T9587, which all carry the recurrent t(3;8)(p21;q12) as the sole anomaly, were selected for molecular analysis, as well as CG682, showing an ins(8;3)(q12;p21.3p14.1), and CG588 which carries a t(8;15)(q12;q14). CG tumors are obtained from Department of Pathology, Göteborg University, Sahlgrenska University Hospital, S-41345 Göteborg, Sweden. The tumor identified as T9587 is obtained from University Hospital Ghent, Belgium. FISH analysis was performed as previously described (Röijer et al. (1996)). Slides were examined in a Zeiss Axiophot epifluorescence microscope using the appropriate filter combinations. Fluorescence signals were digitalized, enhanced and analyzed using the ProbeMaster FISH image analysis system (Perceptive Scientific Instruments, Houston, Texas). Color prints were produced using a Kodak XL 7700 monochrome continuous printer.

### 2.2 Preparation and analysis of DNA and RNA

Extraction of genomic DNA and Southern blot analysis were performed as described previously (Schoenmakers et al. (1994)). Total RNA was extracted from biopsy samples using the TRIZOL (Gibco/BRL) method and Northern analysis was performed according to standard procedures (Sambrook et al. (1989)). DNA from YACs, cosmids, PCR products, and oligonucleotides was labelled using a variety of techniques. For FISH, cosmid clones or inter-Alu PCR products of YACs were biotinylated with biotin-11-dUTP (Boehringer) by nick translation. For filter hybridizations, probes were radio-labelled with a-³²P-dCTP using random hexamers (Feinberg & Vogelstein (1984)). In case of PCR-products of T-genes of the invention smaller than 200 bp in size, a similar protocol was applied, but T-gene specific oligonucleotides were used to prime labelling reactions. Oligonucleotides were labelled using γ-³²P-ATP.

### 2.3 YAC, cosmid, phage, and cDNA libraries

YAC clones in this paper were isolated from the CEPH mark 1 YAC library (Albertsen et al. (1990)), using a combination of PCR-based screening and colony hybridization analysis (Green & Olson (1990)). YAC DNA was isolated and characterized as described before (Schoenmakers et al.(1995); Schoenmakers et al. (1994)). Cosmid clones were isolated from an arrayed human chromosome 8-specific cosmid library (Wood et al. (1992)) obtained from Los Alamos National Laboratory (LANL). LANL-derived cosmid clones are indicated by their unique microtiter plate addresses. Phage clones were derived from a genomic library constructed with Li-14/SV40 DNA (Schoenmakers et al. (1994)) in λFIXII according to standard procedures. Cosmid and phage DNA was extracted using standard techniques involving purification over Qiagen tips (Qiagen). Positive cDNA clones were identified in a mixed poly-dT/random-primed library in λgt11 constructed from human fetal kidney (Clontech). Library screening was performed by plaque hybridization using various DNA probes, derived from subsequently isolated cDNA clones, according to the manufacturer's instructions.

### 2.4 DNA sequencing and computer analysis

Nucleotide sequences were determined according to the dideoxy chain termination method using the T7 polymerase sequencing kit of Pharmacia/LKB or the dsDNA Cycle Sequencing System (GIBCO/BRL). Sequencing results were analyzed using an A.L.F. DNA sequencer™ (Pharmacia Biotech) on standard 30 cm, 6% Hydrolink® Long Range™ gels (AT Biochem). Sequence analysis utilized Lasergene (DNASTAR) and BLAST and BEAUTY searches (NCBI).

### 2.5 PCR amplification of genomic DNA

PCR amplifications were carried out essentially as described before (Schoenmakers (1994)). The following amplimers were used to generate a PLAG1 exon 1 probe (5'-CAA TGG CTG CTG GAA AGA GG-3' and 5'-CCC GTC CGC CGC CTC TAC ACC-3'), a PLAG1 ORF probe (5'-CGT AAG CGT GGT GAA ACC AAA C-3' and AGG GTC GTG TGT ATG GAG GTG A-3'), a PLAG1 3'-UTR probe (5'-ACA TGG CAT TTC GTG TCA CT-3' and 5'-CCA CAA TGG CTC TAG AT-3') and a CTNNB1 exon 1 probe (5'-TGT GGC AGC AGC GTT GGC CCG GC-3' and 5'-CTC AGG GGA ACA GGC TCC TC-3').

### 2.6 Rapid amplification of cDNA ends (RACE)

Rapid amplification of 3' cDNA-ends (3'-RACE) was performed using a slight modification of part of the GIBCO/BRL 3'-ET protocol. For first strand cDNA synthesis, adapter primer (AP2) AAG GAT CCG TCG ACA TC(T)17 was used. For both initial and secondary rounds of PCR, the universal amplification primer (UAP2) CUA CUA CUA CUA AAG GAT CCG TCG ACA TC was used as "reversed primer". In the first PCR round the following specific "forward primers" were used: i) 5'-CAA TGG CTG CTG GAA AGA GG-3' (exon 1) or ii) 5'-AGA ATT TGG GCC TCA GAC AAG ATA-3' (3'-UTR, exon 5). In the second PCR round the following specific forward primers (nested primers as compared to those used in the first round) were used: i) 5'-CAU CAU CAU CAU GGC CGG AGG GAG GAT GTT AA-3' (exon 1) or ii) 5'-CAU CAU CAU CAU ATT GTC CTG GGT TGA TTA TGC AT-3' (3'-UTR, exon 5). CUA/CAU-tailing of the nested, specific primers allowed the use of the directional CloneAmp cloning system (GIBCO/BRL).

For 5'-RACE experiments, the Marathon cDNA Amplification kit (Clontech) was used according the manufacturer's instructions with minor modifications. The 5'-untranslated end of the normal PLAG1 transcript as well as the chimeric transcripts were isolated by 5'-RACE. First strand placenta or adenoma cDNA respectively, was synthesized from 5 µg total RNA using the MV2 primer (5'-CTG CAC TTG ACC CAC CCC TTG GAT-3') located in exon 5. The ds cDNA was ligated to the adaptor and amplified using the anchor primer AP1 and the MV5 primer (5'-CAG GAG AAT GAG TAG CCA TGT GC-3') also located in exon 5. A second round of PCR was performed using the anchor primer and the MV6 primer (5'-TGC ACT TGT AGG GCC TCT CTC CTG-3') located in exon 4. The final PCR products were purified out of agarose gel and cloned into the pCRII vector (Invitrogen).

### 2.7 RT-PCR

Total RNA (5 µg) was reverse-transcribed using Superscript II reverse transcriptase (GIBCO BRL) and oligo d(T) primers according to the recommended conditions. 0.25 µg of the resulting cDNA was subject to amplification using a variety of primer sets. The amplification conditions for the CTNNB1/PLAG1 fusion transcripts were 30 cycles at 94 °C for 10 sec and 68 °C for 1 min in a final volume of 50 µl using the Expand long template PCR system (Boehringer Mannheim). The first round PCR was carried out with the CTNNB1 primer 5'-TGT GGC AGC AGC GTT GGC CCG-3' (CAT-UP) and the PLAG1 primer 5'-CAG GAG AAT GAG TAG CCA TGT GC-3' (MV5). The second round was performed on a 20 fold diluted sample with the CTNNB1 primer 5'-ACG GAG GAA GGT CTG AGG AGC AG-3' (NECAT-UP) and the PLAG1 primer 5'-TGC ACT TGT AGG GCC TCT CTC CTG-3' (MV6). To amplify the reciprocal PLAG1/CTNNB1 fusion transcript two rounds of PCR amplification were performed with 30 cycles at 94 °C for 30 sec, 63 °C for 30 sec and 72 °C for 1 min in a final volume of 50 µl. The first round was carried out with the PLAG1 primer 5'-CAA TGG CTG CTG GAA AGA GG-3' (START-UP) and the CTNNB1 primer 5'-AAG GAG CTG TGG TAG TGG CAC-3' (CAT3). The second round was performed on a 20 fold diluted sample with the PLAG1 primer 5'-GGC CGG AGG GAG GAT GTT AA-3' (START-RACE) and the CTNNB1 primer 5'-GCC GCT TTT CTG TCT GGT TCC A-3' (CAT3NEST).

### 3. Results

### 3.1 The chromosome 8q12 breakpoint cluster region in pleomorphic adenoma

As part of a positional cloning effort to isolate the gene(s) affected by the t(3;8)(p21;q12) in pleomorphic adenomas, we constructed a yeast artificial chromosome (YAC) contig consisting of 34 overlapping YACs, spanning about 2 Mb within band 8q12 (to be published elsewhere). A long range STS and rare cutter physical map of this region was also developed. In previous experiments the t(3;8) breakpoint was mapped to a 1 Mb region flanked by MOS as proximal and by D8S166 as distal marker. One YAC within this region was shown to span the t(3;8) breakpoint in two tumors (CG588 and CG644). To further narrow down the breakpoint region we isolated cosmids corresponding to landmarks within this and other YACs in our contig (Fig. 3A). By FISH, we could demonstrate that the breakpoints in four out of four adenomas tested (CG580, CG588, CG644 and CG682) clustered within a 300 kb subregion located between MOS and STS CH129 (Fig. 3A). Two cosmids from this region, CEM23 and CEM48, were shown to span the breakpoints in adenomas CG644 (Fig. 3B) and CG682 (data not shown), respectively. To refine the distribution of breakpoints within this 300 kb subregion, we developed a contig consisting of 29 phage and cosmid clones (Fig. 3A).

### 3.2 Identification and characterisation of the PLAG1 gene in the 8q12 breakpoint region

While initiating experiments to identify candidate genes mapping within the 300 kb genomic segment harbouring the adenoma breakpoints, BLAST searches (Altschul et al. (1990)) of newly obtained STSs mapping within the 300 kb contig revealed that the right end (CH283) of YAC clone 143D5 displayed sequence identity with a publicly available EST (expressed sequence tag) (GenBank Accession number D59273). Its position in the contig raised the possibility that this EST belonged to the candidate pleomorphic adenoma gene (PLAG1), we were searching for.

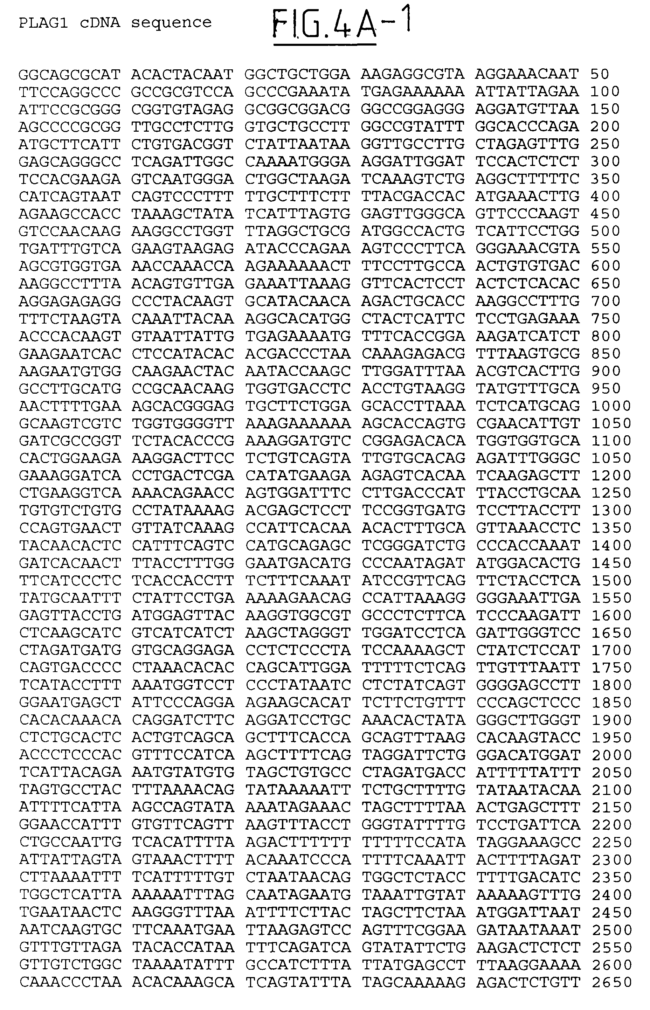

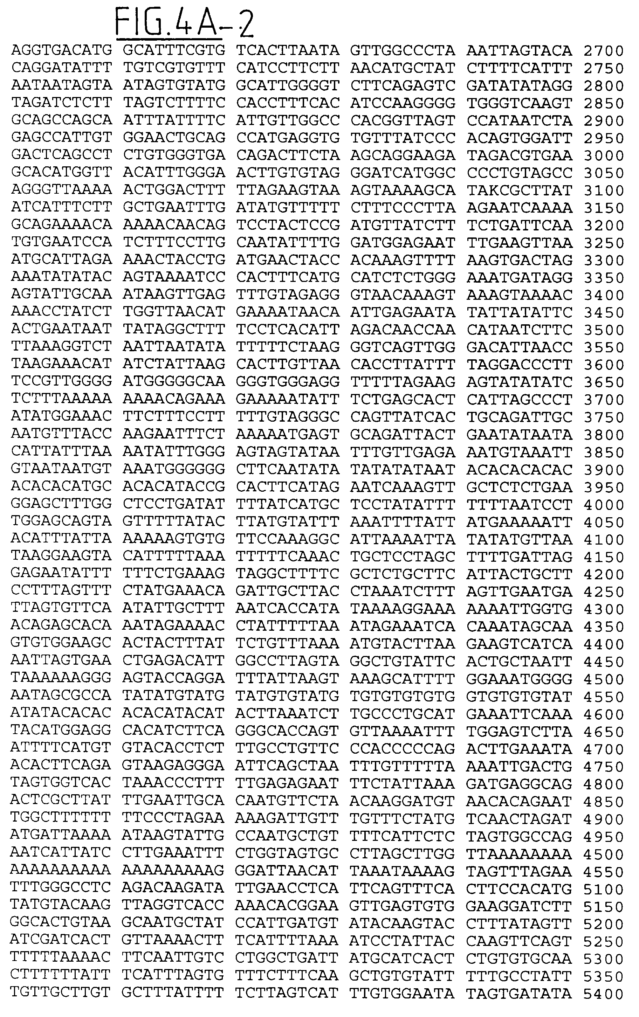

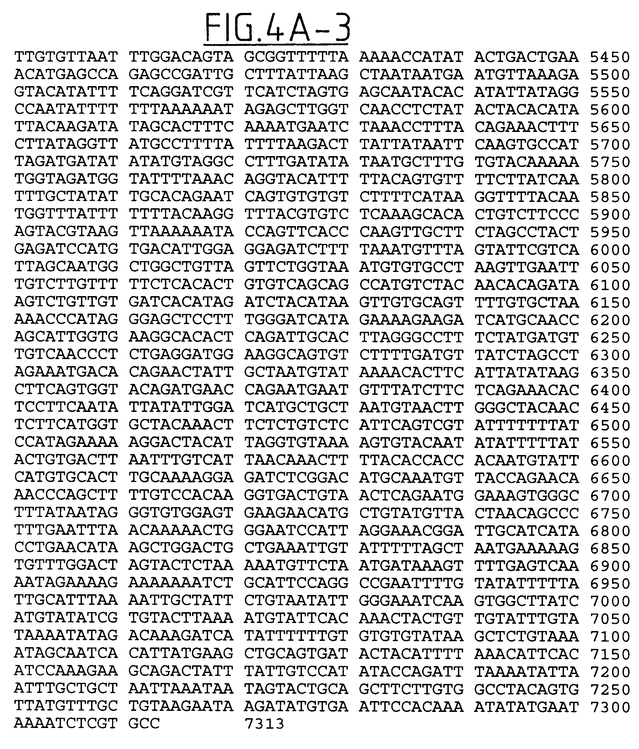

In initial Northern blot experiments using a PCR probe corresponding to this particular YAC end, a 7.5 kb transcript was detected. A combination of sequential screenings of a human fetal kidney cDNA library as well as 5'- and 3'-RACE experiments led to the isolation of a composite cDNA of 7313 nucleotides (GenBank accession number U65002). This cDNA contains an open reading frame (ORF) of 1500 bp starting with the ATG at position 481-483 (Fig. 4A). An in frame stop codon (TAG) is present 9 nucleotides upstream of this ATG. The deduced amino acid sequence reveals seven canonical C2H2 zinc finger domains (Fig. 4B) and a non-finger region of 259 amino acid residues representing the carboxy-terminus of the deduced protein. The zinc finger motifs including the linker sequences are between 28 and 35 amino acids long. The cysteine (C) and histidine (H) residues are present in their characteristic positions in each finger. The typical phenylalanine (F) and leucine (L) residues in finger 4 and 6 are lacking. Strictly spoken, the deduced protein is not a Kruppel zinc finger protein, since it does not contain the characteristic H/C linker (consensus sequence TGEKPYK) in between the zinc fingers (Bellefroid et al. (1989)). Only the seven amino acids between finger 1 and 2 resemble the H/C linker (TGERPYK). The amino-terminal region contains two nuclear localization signals (KRKR and KPRK). The carboxy-terminus is serine-rich (45 amino acid residues out of 259, i.e 17%), raising the possibility of a regulatory role that may be controlled by serine/threonine kinases.

Outside the ORF, some interesting features in the cDNA can be observed. In the 3' untranslated region (UTR), which is 5333 bp long, a polyadenylation signal is present starting at position 7297, and there is also a TG-repeat, which might be of regulatory relevance. In addition, there is an ATTTA sequence motif, which has previously been shown to mediate mRNA destabilization in certain lymphokine and immediate early genes (Sachs (1993)). The ATTTA motif is repeated twelve times in the 3'-UTR.

No sequence similarity was found to any of the known zinc finger proteins but preliminary studies have indicated that the human genome contains at least one additional gene that is closely related to our candidate pleomorphic adenoma gene. Nine anonymous ESTs (expressed sequence tags) with sequence similarity to sequences of our new gene were found in public databases and these ESTs could be aligned resulting in a contiguous sequence of about 1 kb. An open reading frame appeared to be present with coding potential for protein sequences with zinc-finger-like features. Sequence identity between the aligned ESTs and our new gene appeared to be about 75% at the amino acid sequence level; i.e. in the region of our gene encoding zinc fingers 4 to 7. Of interest to note furthermore is that these anonymous ESTs sequences have been mapped to chromosome 6q24, which is implicated in tumors of the salivary glands.

### 3.3 Genomic organization of the PLAG1 gene

Comparison of transcribed and genomic DNA sequences of the PLAG1 gene revealed that it contains 5 exons (Fig. 3A). The transcriptional orientation of the gene is directed towards the centromere. The first three exons are noncoding, the fourth exon contains the translation start site (ATG), and the amino-terminus of the protein including one complete zinc finger domain. The second finger is split by intron 4 and continues into exon 5, which contains the remaining part of the ORF and the long 3'-UTR (5533 bp). Based on our YAC and cosmid maps, the PLAG1 locus spans about 35 kb, with a large intron (approximately 25 kb) between exon 1 and exon 2. 3'-and 5'-RACE analysis revealed the existence of two alternatively spliced mRNAs which differ from each other by the presence or absence of the noncoding exon 2. The difference in electrophoretic mobility of the two mRNA isoforms is too small to distinguish them by Northern blot analysis. The functional relevance of this alternative splicing remains to be elucidated.

### 3.4 Rearrangements of PLAG1 in primary pleomorphic adenoma

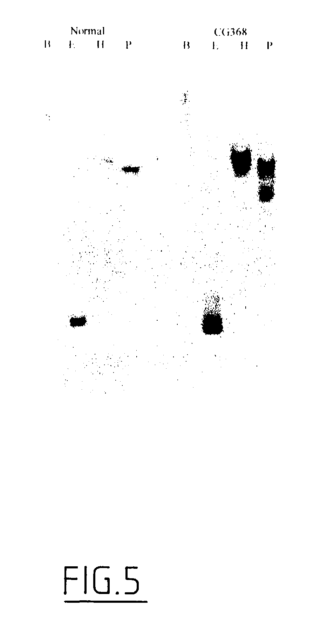

In an attempt to establish whether the 8q12 aberrations in primary pleomorphic adenomas with t(3;8)(p21;q12) led to rearrangements in PLAG1, Southern blot experiments were performed using various PLAG1-derived probes. With a probe within the ORF (STS probe EM265) or the 3'-UTR (KK64) of PLAG1, no rearrangements were detected. However, using probes corresponding to the 5' noncoding region of PLAG1, i.e. intron 1 (STS probes EM416 or EM317) or exon 2 (STS probe EM440), rearrangements were detected in each of the t(3;8) tumors tested (data not shown). Similar Southern blot analysis of primary pleomorphic adenoma CG580, which carries a t(8;15)(q12;q14), revealed also a rearrangement in the 5' noncoding region of PLAG1 (Fig. 5). These results seem to directly implicate PLAG1 as a critical gene in pleomorphic adenoma tumorigenesis. Furthermore, the mechanism might involve transcriptional deregulation of PLAG1, since the rearrangements in PLAG1 were invariably found in the 5' noncoding region of PLAG1 leaving its coding region intact.

### 3.5 t(3;8) results in promoter swapping between PLAG1 and the gene for β-catenin, CTNNB1

The observation that the 8q12 translocation breakpoints in various primary tumors were mapped to intron 1 or intron 2 of the 5'-noncoding region of PLAG1 raised the possibility that the t(3;8) results in the production of a chimeric transcript consisting of PLAG1 sequences fused to those of a gene located on chromosome 3p21. To test this possibility, 5'-RACE experiments were performed using total RNA of the primary tumors CG644 and CG682, which both carry a 3;8-rearrangement. We used a primer specific for exon 5 (MV2) in the cDNA synthesis. Following ligation of an adaptor to the cDNA, PCR amplification was performed using, in the first round, an adaptor-specific primer and a PLAG1-specific primer corresponding to sequences of exon 5 (MV5) and, in a second round, nested adaptor-specific primer and PLAG1-specific primer corresponding to sequences of exon 4 (MV6). Nucleotide sequence analysis of the PCR product revealed that the ectopic sequences were fused to the acceptor splice site of exon 3 of PLAG1. BLAST analysis revealed that they were identical to exon 1 sequences of CTNNB1 (Nollet et al. (1996)), the gene for β-catenin, which has previously been assigned to chromosome 3p21 (Kraus et al. (1994)).

Independent evidence for the involvement of CTNNB1 in the t(3;8)-rearrangements in pleomorphic adenomas was obtained by FISH using two YACs (756G5 and 750D3), which contain the complete gene for β-catenin. According to published maps YAC 756G5 is contained within the proximal part of mega-YAC 750D3 (Gemmill et al. (1995)). As expected, both YACs spanned the 3p21 breakpoint in adenoma CG682, with hybridization signals on the normal 3, the der(3) and the der(8) (data not shown). Detailed analysis of a few prometaphase cells enabled us to sublocalize the β-catenin locus to band 3p21.3, which is in accordance with previous mapping data.

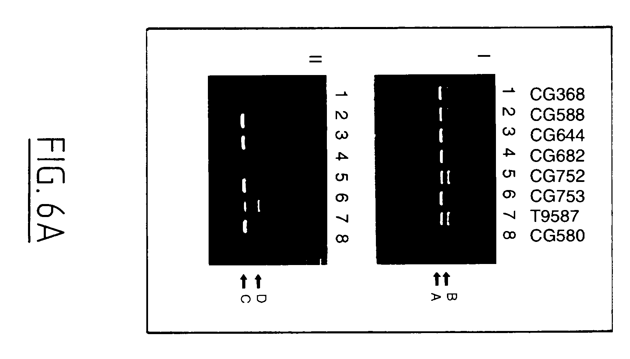

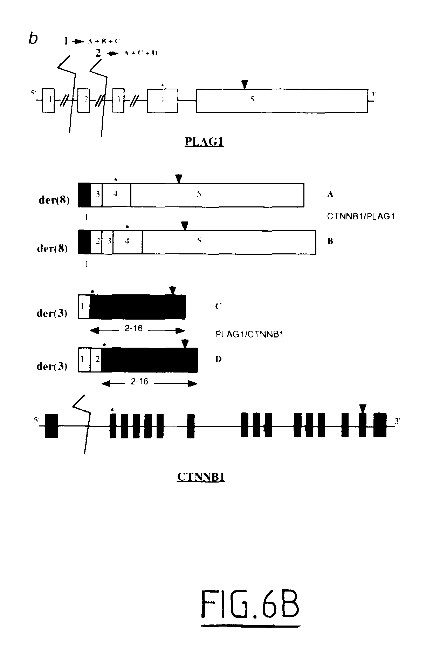

To extend our observation to a larger number of tumors, we applied a reverse transcription-polymerase chain reaction (RT-PCR) approach. RT-PCR amplifications using primers specific for CTNNB1 and PLAG1 were carried out with RNA from tumors CG368, CG588, CG644, CG752, CG753 and T9587, which all carry the recurrent t(3;8), and from tumor CG682, which carries an ins(8;3)(q12;p21.3p14.1). RNA from tumor CG580, which carries a t(8;15), was included as a negative control. PCR experiments resulted in the generation of PCR products corresponding to hybrid transcripts consisting of PLAG1 and CTNNB1 sequences, in seven out of seven t(3;8) tumors analyzed (Fig. 6). In tumors CG368, CG588, CG682, CG752, and T9587, PCR products of 509 bp (Fig. 6A, PCR product A) and 614 bp (Fig. 6A, PCR product B) were generated, whereas in tumors CG644 and CG753, only the PCR product of 509 bp was found. The PCR product of 509 bp (from NECAT-UP up to MV6) is corresponds to a hybrid transcript containing exon 1 of CTNNB1 and exons 3 to 5 of PLAG1. The PCR product of 605 bp contains an extra 105 bp, which corresponds to the alternatively spliced exon 2 of PLAG1. It points towards the presence of a related isoform consisting of exon 1 of CTNNB1 and exons 2 to 5 of PLAG1. This was also confirmed by nucleotide sequence analysis of the PCR products. We were also able to demonstrate that the corresponding reciprocal fusion transcripts are expressed. In all tumors except CG682, a PCR product of 130 bp was generated corresponding to a fusion transcript consisting of exon 1 of PLAG1 and exons 2 to 16 of CTNNB1. In tumor CG753, an additional PCR product was detected, corresponding to a fusion transcript consisting of exons 1 to 2 of PLAG1 and exons 2 to 16 of CTNNB1. This additional band was also observed but with weak intensity in tumor CG644. All these results indicate that in CG644 and CG753, the 8q12 translocation breakpoints are located in intron 2, whereas the breakpoints of tumors CG368, CG588, CG682, CG752, and T9587 are located in intron 1. Interestingly, using 5' RACE analysis with the tumor CG580, which carries a t(8;15) translocation, we found that the breakpoint occurs also in the same region leading to a fusion transcript with ectopic fused to exon 3 of PLAG1.

### 3.6 Activation of PLAG1 expression due to promoter swapping

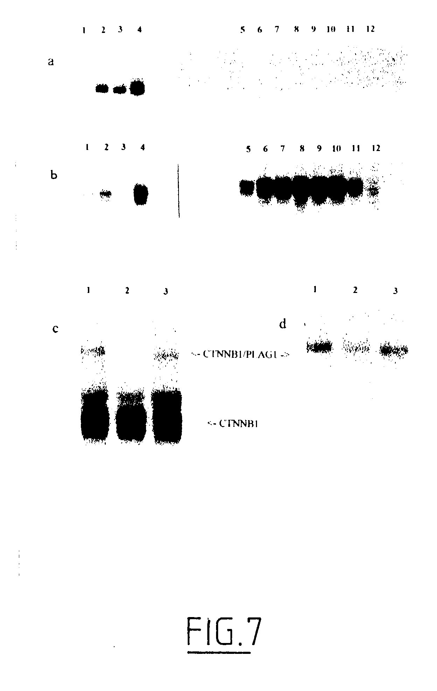

Northern blot analysis was performed to evaluate the effect of the t(3;8)(p21;q12) translocation on the expression levels of PLAG1 and CTNNB1 (Fig. 7). As previously mentioned, PLAG1 is expressed as a 7.5 kb transcript, which was readily detected in human fetal lung, liver, and kidney but not in fetal brain. In adult tissues, the 7.5 kb transcript was not detected in human heart, brain, lung, liver, skeletal muscle, kidney, pancreas, and salivary gland. Low levels of PLAG1 expression was found in human placenta (Fig. 7A). In contrast, the CTNNB1 gene was ubiquitously expressed as a 3.8 kb RNA doublet in all tissues tested (Fig. 7B). Similar results were obtained in the Northern evaluation of fetal and adult mouse tissues (data not shown).

Northern blot analysis was performed on two pleomorphic adenomas with t(3;8) and on one tumor with a variant t(8;15) (Fig. 7C). In contrast to normal salivary gland tissue in which no PLAG1 transcripts could be detected, the three tumors expressed a 7.5 kb transcript. This transcript does not correspond to the normal PLAG1 transcript (exons 1 to 5), since an exon 1-specific probe failed to hybridize to this transcript (data not shown). Instead, a probe specific for exon 1 of CTNNB1 recognized this 7.5 kb transcript as well as the 3.8 kb doublet of CTNNB1 transcripts in the two tumors with a t(3;8). In contrast, only the 3.8 kb doublet of CTNNB1 transcripts was detected in adenoma CG580 with a variant t(8;15). The expression level of CTNNB1 in this tumor was about twofold higher as compared to tumors CG644 and CG682, which is expected since none of the CTNNB1 alleles is rearranged. Collectively, these results suggest that PLAG1 expression in these pleomorphic adenomas is driven by the 5' regulatory sequences either of the CTNNB1 gene for the cases with t(3;8) or of an as yet unknown gene on chromosome 15 for the case with t(8;15). These results indicate that the principal mechanism in pleomorphic adenomas with 8q12 rearrangements is activation of PLAG1 expression, due to promoter swapping between PLAG1 and a translocation partner gene, preferentially CTNNB1. Furthermore, expression of the translocation partner gene is likely to be down-regulated concomitantly; e.g. as demonstrated for CTNNB1.

### 4. Discussion

We have characterized a novel gene, PLAG1, which we propose is a critical locus involved in the development of pleomorphic adenoma of the salivary glands. The gene was identified on the long arm of chromosome 8, close to the MOS proto-oncogene, as a result of a positional cloning project to molecularly define the t(3;8)(p21;q12), which is the most frequent chromosome aberration in pleomorphic adenomas. We have also identified the translocation partner gene at 3p21, i.e. the CTNNB1 gene which encodes β-catenin. In addition to 8q12 and 3p21, chromosome rearrangements involving 12q13-15 are also frequently observed in pleomorphic adenomas. The high mobility group protein gene HMGIC was recently identified as the gene consistently rearranged in these cases (Schoenmakers et al. (1995)). In conclusion, we have molecularly defined the three main chromosomal rearrangements implicated in the genesis of pleomorphic adenomas.

Structural rearrangement of the 8q11-13 region are consistently found also in benign lipoblastomas. These tumors are considered to be unique variants of lipoma, occurring primarily in children before 3 years of age. Among the seven cases so far analyzed cytogenetically, rearrangements involving 8q11-13 were observed in six cases (Mitelman (1994)). Similar to pleomorphic adenomas, the translocation partners of 8q12 are variable. The similarities in chromosomal pattern between these two tumor types suggest that PLAG1 is the target of the 8q11-13 rearrangements also in lipoblastomas.

We have also elucidated the molecular consequences of the t(3;8) translocation. The 8q12 translocation breakpoints were invariably found in the 5'-non-coding region of PLAG1 in all seven primary tumors tested. The breakpoints in the CTNNB1 gene were always found in the first intron. As a result of the translocation, the coding sequences of PLAG1 are brought under control of the 5' regulatory sequences of the CTNNB1 gene, and vice versa, resulting in the activation of PLAG1 and down-regulation of CTNNB1. Based on their expression patterns in normal fetal and adult tissues, it is clear that the regulation of expression of the two genes is very different. The CTNNB1 gene is highly and ubiquitously expressed, whereas PLAG1 is developmentally regulated, with expression restricted to fetal tissues. It should be emphasized that in adult tissues, PLAG1 is either not expressed or expressed at very low levels, In the adenomas, both the CTNNB1/PLAG1 and the reciprocal PLAG1/CTNNB1 transcripts were detected by RT-PCR; detection of the latter transcripts sometimes required three rounds of PCR, indicating that the expression levels are very low. Our findings represent the first example of reciprocal exchange of expression control elements in solid tumors. Since the coding sequences of both genes are invariably preserved, the molecular mechanism could be classified as promoter swapping, resulting in activation of PLAG1 and down-regulation of CTNNB1 expression.

Since the t(3;8) in pleomorphic adenomas invariably leads to activation of PLAG1, it is of interest to evaluate possible physiological implications. The PLAG1 gene encodes a protein with a deduced molecular weight of 56 kDa and a deduced pI of 8.56. Analysis of the open reading frame of PLAG1 reveals seven zinc fingers in the amino-terminal region. The carboxy-terminal region is rich in serine residues. Furthermore, two potential nuclear localization signals are present (residues 22-25 and 29-32). Collectively, this suggests that the PLAG1 protein is a novel member of the large zinc finger gene family. Zinc finger motifs were originally identified as DNA binding structures in the RNA polymerase III transcription factor TFIIIA, which binds to the internal control region of the 5S RNA gene. TFIIIA-like zinc fingers are present in a variety of regulatory proteins found in higher and lower eukaryotes. This type of zinc finger motif consists of ≈30 amino acids with two cysteine and two histidine residues (C2H2) that stabilise the domain by tetrahedrally coordinating a Zn²⁺ ion. A region of ≈12 amino acids between the invariant cysteine-histidine pairs is characterized by scattered basic residues and several conserved hydrophobic residues. Most zinc finger genes studied so far encode polymerase II transcription factors. Apart from transcriptional modulation and control of RNA metabolism, chromatin packaging might also constitute an important activity through which zinc finger proteins exert their regulatory roles (El-Baradi & Pieler (1991)). It should also be noted that the presumed role of HMGIC is also in chromatin modelling (Schoenmakers et al. (1995); Ashar et al. (1995); Wolffe (1994)). The mammalian genome is known to contain a large number of C2H2 zinc finger genes (Bellefroid et al. (1989)) and the number of such genes implicated in cancer is growing steadily.

The function of the serine rich carboxy-terminal part of PLAG1 is unknown but it may have a regulatory function that can be controlled by serine/threonine kinases. If PLAG1 encodes a DNA binding protein, as the presence of its zinc fingers suggests, the carboxy-terminal region might represent the transactivation domain. At least four different primary sequence motifs that characterize the activation domains are identified thus far, i.e. acidic, glutamine-rich, proline-rich and serine/threonine-rich. They likely represent regions that functionally interact with other proteins (Mitchell & Tjian (1989)).

Activation of PLAG1 due to promoter substitution may lead to deregulation of genes normally controlled by PLAG1. If overexpression of PLAG1 is indeed a major molecular consequence of the 8q12 translocations than this could possibly be achieved also by other mechanisms, including for instance an increase in gene copy number. In this context it should be noted that a small subgroup of pleomorphic adenomas show trisomy 8, often as the sole anomaly (Mitelman (1994)). In addition, trisomy 8 is a common numerical abnormality found in several histological subtypes of malignant salivary gland tumors as well as in different subtypes of leukemias and sarcomas (Mitelman (1994)).

The preferential fusion partner of PLAG1 is the CTNNB1 gene, which encodes β-catenin, a cytoplasmic protein of about 88 kDa (Gumbiner & McCrea (1996)). Its frequent involvement in pleomorphic adenomas as found here might point towards a critical role of CTNNB1. Most likely, a role of CTNNB1 is to provide an active promoter in front of the PLAG1 gene. However, since the observed promoter swapping between PLAG and CTNNB1 leads to down-regulation of CTNNB1 expression, it may also lead to other physiological consequences, especially since β-catenin is a broad-range protein interface, as that has been implicated in highly diverse processes, such as human colon cancer, epithelial cell adhesion, embryonal axis formation in Xenopus, and pattern formation in Drosophila (Peifer (1993)). The β-catenin protein has also been found as structural component of adherens junctions (AJs) (Peifer (1993); Kemler (1993); Gumbiner (1996)), which are multiprotein complexes assembled around Ca²⁺-regulated cell adhesion molecules (cadherins). β-Catenin binds directly to cadherins and acts as a protein interface between cadherin and the cytoskeleton. The cadherin-β-catenin complex mediates cell adhesion, cytoskeletal anchoring, and signalling, which are important processes for regulation of cell growth and behaviour. It has been suggested that AJ complexes may play a role in the transmission of signals for contact inhibition (Peifer (1993)). Down-regulation of β-catenin, as occurs in pleomorphic adenomas, could interfere with this transmission and lead to growth deregulation. Since β-catenin levels apparently are important physiologically, it might be that down-regulation of CTNNB1 expression plays some role in pleomorphic adenoma development, possibly synergistically to PLAG1. In conclusion, as activation of PLAG1 is observed in all pleomorphic adenomas with 8q12 involvement tested, this seems to constitute a major factor in salivary gland tumor formation. The critical role of down-regulated levels of β-catenin remains to be established.

Previous studies of HMGIC have shown that rearrangements of this gene preferentially occur in particular cytogenetic subgroups of benign mesenchymal tumors, with the only exception so far being a subgroup of pleomorphic adenomas (Schoenmakers et al. (1995)). When considering the findings in the latter type of tumors, it should be noted that, although they are epithelial in origin, they often also show a varying degree of mesenchymal differentiation. This may be explained by the fact that pleomorphic adenomas are thought to originate from a pluripotent reserve cell in the terminal duct system which possesses the capacity to differentiate into both epithelial and myoepithelial cells (Evans & Cruickshank (1970)). The latter cells may function as facultative mesenchymal cells, and are responsible for the production of extracellular material, including myxochondroid stroma (Dardick et al. (1991)). An intriguing question in this context is of course whether HMGIC is preferentially affected in adenomas with a prominent stromal component while PLAG1 is preferentially affected in tumors with little or no stroma. Histological re-examination of all but one of the adenomas with PLAG1 involvement in this study, revealed that all cases contained mesenchymal components, including both myxoid and chondroid tissues, suggesting that mesenchymal differentiation is not restricted to adenomas with HMGIC involvement. It should be noted that previous cytogenetic observations have already indicated that chromosome 12q13-15 and 8q12 rearrangements constitute mutually exclusive pathways for the evolution of pleomorphic adenomas (Sandros et al. (1990)).

The identification and cloning of a novel "benign oncogene" activated by chromosome translocations constitute a major step towards an increased understanding of the molecular pathogenesis of benign neoplastic growth. Our findings may lead to new insights into the genetic differences between benign and malignant tumors. In addition, the present findings may have diagnostic implications. The fact that PLAG1 is not normally expressed in adult tissues, but activated in pleomorphic adenomas due to 8q12 rearrangements, makes it a potentially useful tumor marker in the differential diagnosis of benign and malignant salivary gland tumors. On the basis of the fusion transcripts that are formed, highly specific diagnostic assays can be developed, as will be illustrated in the following examples.

### FIGURE LEGENDS

### Figure 3

**A:** Contig of 3 overlapping YACs (bold lines), 27 cosmids and 2 phages, containing 27 landmarks and spanning a 300 kb DNA region on chromosome 8q12. Contig elements are labelled or numbered and defined in the list below. Cosmid clones isolated from the arrayed chromosome 8-specific cosmid library constructed at Los Alamos National Laboratory (LANL) (Wood et al. (1992)) are named after their unique microtiter plate addresses. #1 and #22 are genomic phage clones; #21 is a clone isolated from the non-arrayed LANL chromosome 8-specific cosmid library. The orientation of the contig on the long arm of chromosome 8 is given as well as the order of 27 landmarks. It should be noted that the contig is not scaled. Below the contig, the genomic organization and the relative location of the PLAG1 gene is given schematically, with exact sizes (bp) of its exons and estimated sizes (kb) of its introns. Noncoding sequences are represented as open boxes and coding sequences as black boxes. The relative positions of the translation initiation (ATG) and stop (TAG) codons in PLAG1 are indicated. At the bottom of the figure, characteristics of the deduced protein encoded by PLAG1 are given. Zinc fingers are labelled F1-F7.

| | | |
|---|---|---|
| 1 = PEM18 ← CH33 =D8SXXX | 11 = 163E1 ← CH280 =D8SXXX | 21 = CEM55 ← EM195 =D8SXXX |
| 2 = 96E9 ← PENK =D8SXXX | 12 = 203B8 ← CH280 =D8SXXX | 22 = PEM49 ← EM317 =D8SXXX |
| 3 = 41E12 ← PENK =D8SXXX | 13 = 50B7 ← EM187 =D8SXXX | 23 = 149G12 ← D8S285 |
| 4 = 17B6 ← EM172 =D8SXXX | 14 = 115H9 ← EM187 =D8SXXX | 24 = 64C1 ← D8S285 |
| 5 = 93B2 ← EM172 =D8SXXX | 15 = 129B10 ← CH129 =D8SXXX | 25 = 64D1 ← D8S285 |
| 6 = 47D11 ← CH280 =D8SXXX | 16 = 116A2 ← CH129 =D8SXXX | 26 = 143B1 ← MOS =D8SXXX |
| 7 = 53B10 ← CH280 =D8SXXX | 17 = 7B7 ← CH129 = D8SXXX | 27 = 143H1 ← MOS =D8SXXX |
| 8 = 66E5 ← CH280 =D8SXXX | 18 = 4H4 ← EM156 =D8SXXX | 28 = 148E9 ← MOS =D8SXXX |
| 9 = 144A7 ← CH280 =D8SXXX | 19 = 4H5 ← EM156 =D8SXXX | 29 = 22B8 ← MOS =D8SXXX |
| 10 = 163D11 ← CH280 =D8SXXX | 20 = 4H6 ← EM156 =D8SXXX | |
| (clone number, cosmid or phage clone, STS, accession number) | | |

**B:** Mapping of the 8q12 translocation breakpoint in an adenoma (CG644) with a t(3;8)(p21;q12). Cosmid CEM48 (white spots) was co-hybridized with an alpha-satellite probes one specific for chromosome 3 and the other for chromosome 8 (black spots). Hybridization signals were found on the normal 8, the der (3), and the der (8), indicating that CEM48 spans the t(3;8)(p21;q12) breakpoint. Chromosomes are stained in blue with DAPI.

### Figure 4

**A:** cDNA and deduced amino acid sequence of PLAG1. The relative positions of exon/intron boundaries are indicated by triangles ( 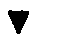 ). The conserved C, F, L and H residues in the zinc finger domains are underlined. Residues 22-25 and 29-32 constitute two potential nuclear localization signals. A potential polyadenylation signal and the putative mRNA destabilizing ATTTA motifs in the 3'-noncoding region are underlined. The nucleotide sequence of the complete cDNA has been deposited at GenBank under accession number XXX.
**B:** Alignment of the seven zinc-finger-like motifs found in the deduced amino acid sequence of PLAG1 relative to the C2H2 consensus motif. The canonical C, F, L and H residues are given in bold.

### Figure 5

Illustrative example of the detection of DNA rearrangement in PLAG1 of a primary pleomorphic adenoma using Southern blot analysis. DNA of pleomorphic adenoma CG368 (panel I) and control DNA isolated from normal lymphocytes (panel II) were digested with restriction endonuclease BamHI (B), EcoRI (E), HindIII (H), or PstI (P), as indicated. A probe with specificity for exon 3 of PLAG1 (EM440) was used. The molecular weight markers are 23.1, 9.4, 6.5, 4.3, 2.3 and 2.0 kb, respectively.

### Figure 6

**A:** Detection of CTNNB1/PLAG1 and PLAG1/CTNNB1 fusion transcripts by RT-PCR in primary adenomas. **I.** CTNNB1/PLAG1 were detected using the RT-PCR protocol and primers described in detail in Methods. Primary tumors analyzed included CG368 (lane 1), CG588 (lane 2), CG644 (lane 3), CG682 (lane 4), CG752 (lane 5), CG753 (lane 6), T9587 (lane 7), and CG580 (lane 8). **II.** PLAG1/CTNNB1 fusion transcripts were detected similarly using the same samples as under "**I**'. Details of the primers used here are given in Methods Section. PCR products are labelled A-D.
**B:** Schematic representation of the nature and origin of CTNNB1/PLAG1 and PLAG1/CTNNB1 fusion transcripts in primary pleomorphic adenomas with t(3;8)(p21;q12). At the top of the figure, the exon/intron distribution of the PLAG1 gene is given, at the bottom, the exon/intron distribution for the CTNNB1 gene. Positions of chromosome breakpoints are indicated by an arrow ( 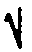 ). Translation initiation sites are indicated by asterisks (*) and stop codons by triangles ( 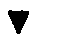 ). A-D: schematic exon compositions of hybrid transcripts, as established by 5'-RACE analysis. A: cDNA sequence junction between exon 1 of CTNNB1 and exons 3-5 of PLAG1. B: cDNA sequence junction between exon 1 of CTNNB1 and exons 2-5 of PLAG1. C: cDNA sequence junction between exon 1 of PLAG1 and exons 2-16 of CTNNB1. D: cDNA sequence junction between exons 1-2 of PLAG1 and exons 2-16 of CTNNB1.

### Figure 7

**A:** Northern blot analysis of the expression pattern of PLAG1 in normal human fetal tissues including brain (1), lung (2), liver (3), kidney (4) as well as adult tissues including heart (5), brain (6), placenta (7), lung (8), liver (9), skeletal muscle (10), kidney (11), and pancreas (12).
**B:** Northern blot analysis of the expression pattern of the CTNNB1 gene in normal human fetal and adult tissues as described under "**A**".
**C:** Detection of CTNNB1/PLAG1 transcripts in pleomorphic adenomas by Northern blot analysis using exon 1 of CTNNB1 as a molecular probe, Lane 1, RNA of CG644 (t(3;8)), lane 2, RNA of CG580 (t(8;15)), and lane 3, RNA of CG682 (ins 3p21 (8q12)). The CTNNB1/PLAG1 fusion transcript is indicated.
**D:** Using the same blot as under "**C**",detection of CTNNB1/PLAG1 transcripts using a probe with specificity for the 3' UTR of PLAG1 (probe KK64). The CTNNB1/PLAG1 transcript is indicated.

### EXAMPLE 3

The discovery of PLAG1 made it possible to identify PLAG1-related genes. The possibility that the human genome contains such genes was raised by Southern blot data showing bands (weak hybridization signals). In this example, the identification of another member of the PLAG gene family that might be of pathogenetical relevance is described, i.e. the PLAG2 gene.

To isolate cDNA clones corresponding to other members of the PLAG1 gene family, a cDNA library in λZAP was used (Stratagene). As molecular probe, the complete human PLAG1 cDNA was used. Screening of the cDNA library was performed according to routine procedures using low stringency hybridization conditions (Schoenmakers et al. (1994)). This resulted in the isolation of a number of overlapping cDNA clones presumably corresponding to another member of the PLAG family. From the inserts of these, a 2.7 kbp composite cDNA could be constructed that contained an open reading frame (1233 nucleotides) for a protein (411 amino acids) structurally similar to PLAG1. This protein was designated PLAG2. The 2.7 kbp human PLAG2 cDNA fragment started 176 nucleotides upstream of the ATG start codon.

The nucleotide sequence of the composite PLAG2 cDNA and the deduced amino acid sequence of the corresponding PLAG2 protein are shown in Figure 8. Nucleotide sequences were determined according to the dideoxy chain termination method using the T7 polymerase sequencing kit of Pharmacia/LKB. DNA fragments, subcloned in the pBLUESCRIPT or pGEM-3Zf(+) vector, were sequenced using standard primers and primers synthesized based upon newly obtained sequences. Nucleotide sequence data were obtained from both strands and analyzed using the sequence analysis computer programs Genepro (Riverside Scientific), PC/Gene and Intelligenetics (IntelliGenetics, Inc.).

To established that the isolated PLAG2 sequences corresponded to expressed sequences, Northern blot analysis was performed according to routine procedures. Total RNA was extracted from various human tissues and cell lines using Trizol™ (GIBCO/BRL). After precipitation, concentrations of RNA in the samples were estimated via spectroscopic determination. 40 µg of total RNA from each sample was run on 1% agarose gels containing 1 x MOPS (0.02 M MOPS (Sigma) pH 7.0, 50 mM Na-acetate, 10 mM EDTA pH 8.0), and 0.66 M formaldehyde (Sigma) in a 1 x MOPS buffer for 16 h at 4 V/cm. After gel electrophoresis, RNA was transferred to Hybond N⁺ filters (Amersham) using 10 x SSPE (20 x SSPE: 3.0 M NaCl, 0.16 M NaH₂PO₄ pH 7.4, 0.02 M EDTA) for standard capillary blotting. After completion of the transfers, nylon filters were irradiated with ultraviolet light using a Stratalinker (Stratagene). Prehybridizations of the blots were carried out for 3 h at 42 °C in hybridization buffer consisting of 5 x SSPE, 10 x Denhardt's, 2% SDS, 50% formamide and 100 µg/ml herring sperm DNA. For hybridizations, blots were incubated overnight in the same buffer as described for the prehybridizations. Subsequently, blots were washed for 40 min at room temperature in 2 x SSC containing 0.05% SDS and for 40 min in 0.1 x SSC containing 0.1% SDS at 50 °C. Autoradiographs were obtained by exposing Kodak X-AR film at -80 °C with intensifying screen.

In Northern blot analysis using the complete 2.7 kb PLAG2 cDNA as molecular probe and rather stringent hybridization conditions, four distinct mRNAs were detected. These varied in size and included mRNAs of about 4.4 kb, 3.1 kb, 2.9 kb, and 1.9 kb. The 3.1 and 2.9 kb transcripts were preferentially seen in fetal tissue, whereas the other transcripts were seen preferentially in adult tissues. These results established the expression of the PLAG2 sequences in a variety of cell types. Furthermore, the detection of various mRNAs with the PLAG2 cDNA probe suggests the existence of various isoforms and raises the possibility that various PLAG2 protein isoforms may be produced.

### EXAMPLE 4

### Detection of hybrid PLAG1 in salivary gland tumor cells.

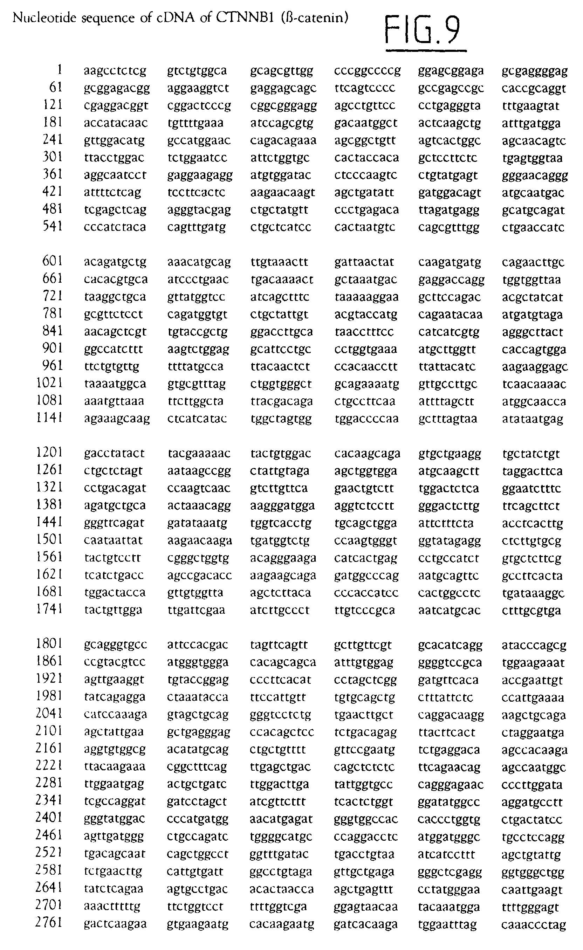

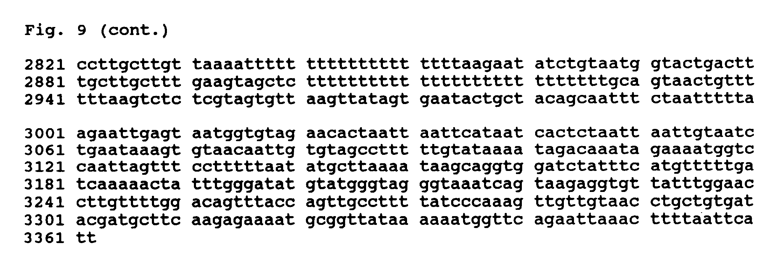

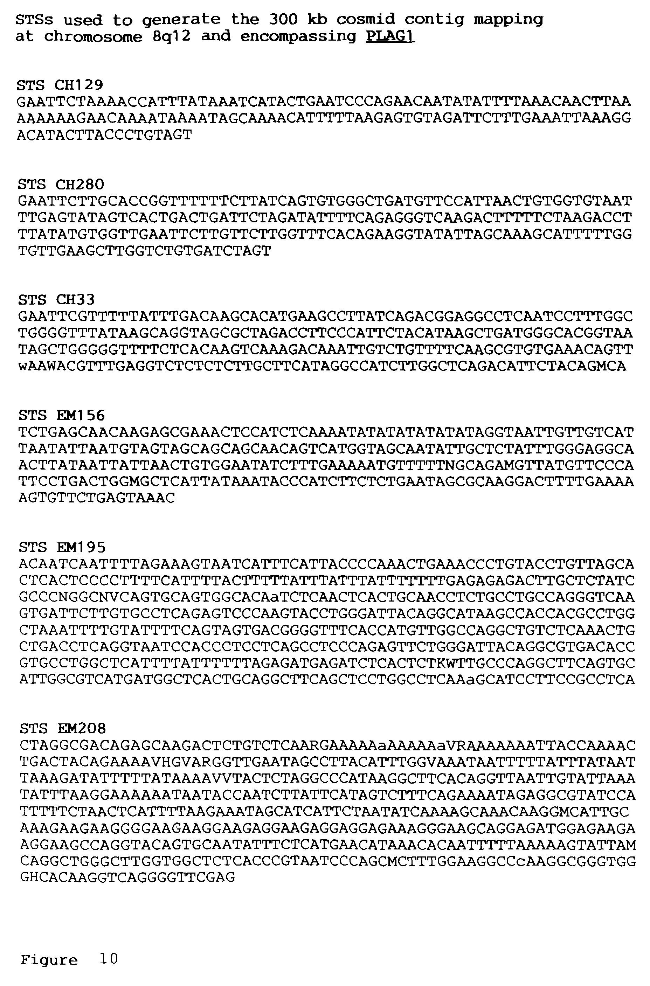

cDNA clones of the chromosome 3-derived CTNNB1 gene were isolated and the nucleotide sequence thereof established. The nucleotide sequence data of a composite cDNA are shown in Fig. 9. The amino acid sequence of the CTNNB1-encoded protein was deduced.

In 5'-RACE analysis of primary pleomorphic adenomas of the salivary glands, PLAG1 containing fusion trancripts were identified. 5'-RACE experiments were performed using total RNA of the primary tumors. A primer specific for exon 5 (MV2) was used in the cDNA synthesis. Following ligation of an adaptor to the cDNA, PCR amplification was performed using, in the first round, an adaptor-specific primer and a PLAG1-specific primer corresponding to sequences of exon 5 (MV5) and, in a second round, nested adaptor-specific primer and PLAG1-specific primer corresponding to sequences of exon 4 (MV6). Nucleotide sequence analysis of the PCR product revealed that the ectopic sequences were fused to the acceptor splice site of exon 3 of PLAG1. BLAST analysis revealed that they were identical to exon 1 sequences of CTNNB1, the gene for β-catenin, which has previously been assigned to chromosome 3p21.

### EXAMPLE 5

### Diagnostic test for pleomorphic adenoma of the salivary glands

Fine needle biopsies of patients having a pleomorphic adenoma of the salivary gland (with a chromosome 8q12 aberration others with a normal karyotype) were taken, From the material thus obtained total RNA was extracted using the standard TRIZOL™ LS protocol from GIBCO BRL as described in the manual of the manufacturer. This total RNA was used to prepare the first strand of cDNA using reverse transcriptase (GIBCO/BRL) and an oligo dT(17) primer containing an attached short additional nucleotide stretch. The sequence of the primer used is as described in Example 2, under point 2.6 [AAG GAT CCG TCG ACA TC(T)17]. RNase H was subsequently used to remove the RNA from the synthesized DNA/RNA hybrid molecule. PCR was performed using a gene-specific primer (Example 2, point 2.6) and a primer complementary to the attached short additional nucleotide stretch. The thus obtained PCR product was analyzed by gel electrophoresis. Fusion constructs were detected by comparing them with the background bands of normal cells of the same individual.

In an additional experiment, a second round of heminested PCR was performed using one internal primer and the primer complementary to the short nucleotide stretch [AAG GAT CCG TCG ACA TC(T)17]. The sensitivity of the test was thus significantly improved.

### EXAMPLE 6

### Diagnostic test for uterine leiomyoma.

Uterine leiomyoma is the most common pelvic tumor in women, occurring with an incidence up to 77% of women of reproductive age when tumors are counted after 2 millimeter serial sectioning of consecutive hysterectomy specimens. Often multiple leiomyomas are present, with estimates as high as an average of 6.5 tumors per uterus. Although most patients with these steroid-dependent tumors are asymptomatic, symptomatic leiomyomas can be associated with abnormal uterine bleeding, pelvic pain, urinary dysfunction, spontaneous abortions, premature delivery and infertility. The high incidence of this benign smooth muscle tumor constitutes a major public health problem with the diagnosis of myomatous uterus leading to over 200,000 hysterectomies performed annually in the United States. Furthermore, non-random inactivation of the X chromosome has clearly demonstrated that uterine leiomyomas are monoclonal proliferations. Although leiomyomata typically are benign tumors, in a very low percentage (< 0.1%) they might be able to metasta-size or even progress to malignancy.

Besides a normal karyotype which is being found in approximately 70% of the cases investigated, several cytogenetically abnormal subgroups, can be distinguished among leiomyomata. Leaving out of consideration the group which shows random changes, one of the largest cytogenetic subgroups (comprising approximately 25% of the cytogenetically abnormal tumors) was first described in 1988 and is characterized by the involvement of 12q15 and/or 14q23-24, mainly as t(12;14)(q14-15;q23-24). Another subgroup, with a similar incidence, contains deletions involving the long arm of chromosome 7, with region q21-22 being the most probable commonly involved region. Another subset of leiomyomas is characterized by numerical aberrations, mainly trisomy 12. This trisomy is found in approximately 10% of the cytogenetically abnormal leiomyomas. Furthermore, chromosome 6p21-pter, has been found to be recurrently involved in roughly 5% of the cases studied. Finally, a small percentage (approx. 3.5%) of leiomyomata shows t(1;2)(p36;p24). In uterine leiomyomata with chromosome 12q14-15 aberrations, the HMGIC gene is affected and in those with chromosome 6p aberrations, the HMGIY gene is affected.

The observation that 70% of the uterine leiomyomata tested show a normal karyotype prompted studies to investigate whether activation of a PLAG gene could be found in uterine tumors. Therefore, Northern blot and PCR analysis were performed.

Specimens of tumor material surgically removed from patients and classified by routine pathology as uterine leiomyoma, uterine leiomyosarcoma, fibroleiomyoma, or uterine fibroma were used. From the material thus obtained, total RNA was extracted using the standard TRIZOL™ LS protocol from GIBCO BRL as described in the manual of the manufacturer. This total RNA was used in Northern blot analysis, which revealed activation of PLAG1 in about 50% of the tumors tested, indicating that specific PLAG activation is also observed in these types of tumors. These results were confirmed by PCR analysis. These results reveal the possibilities of using PLAG1 as a diagnostic marker on routine biopsies taken from patients. Since PLAG1 is implicated in this type of tumor, PLAG1 can be used as a target for the development of novel therapeutic strategies for uterine leiomyoma.

### EXAMPLE 7

### Diagnosis of lipoblastoma.

Lipoblastomas are benign pediatric tumors that are assumed to result from proliferation of primitive adipocytes. These type of tumors often grow rapidly but there are no reports that they metastasize. Because of the rapid growth and their histopathological features, lipoblastomas can mimic myxoid or well-differentiated liposarcoma which makes the diagnosis problematic. As a direct consequence, therefore, some of these tumors have been treated in an unnecessarily agressive manner.

A recurrent rearrangement involving the long arm of chromosome 8 has been reported for lipoblastomas. Southern blot analysis detected a rearrangement in the PLAG1 gene opening the possibility of using PLAG1 as a diagnostic marker.

A biopsy of a patient having a lipoblastoma was taken. From the material thus obtained total RNA was extracted using the standard TRIZOL™ LS protocol from GIBCO BRL as described in the manual of the manufacturer. This total RNA was used to prepare the first strand of cDNA using reverse transcriptase (GIBCO/BRL) and an oligo dT(17) primer containing an attached short additional nucleotide stretch. The sequence of the primer used is as described in Example 2, under point 2.6. RNase H was subsequently used to remove the RNA from the synthesized DNA/RNA hybrid molecule. PCR was performed using a gene-specific primer (Example 2, point 2.6) and a primer complementary to the attached short additional nucleotide stretch. The thus obtained PCR product was analysed by gel electrophoresis. Fusion constructs were detected by comparing them with the background bands of normal cells of the same individual.

In an additional experiment, a second round of hemi-nested PCR was performed using one internal primer and the primer complementary to the short nucleotide stretch. The sensitivity of the test was thus significantly improved.

### EXAMPLE 8

### PLAG2 as diagnostic marker.

To evaluate the diagnostic potential of the PLAG2 gene, we have first identified on which human chromosome the PLAG2 gene is located and subsequently established its subchromosomal location. Such mapping studies could link the gene to known tumor-specific chromosome anomalies. Using several different PLAG2 containing YACs and cosmids in FISH analysis, the PLAG2 gene was tentatively mapped to chromosome band 6q24, a chromosome segment implicated in various tumors such as for instance malignant salivary gland tumors.

Malignant salivary gland tumors are a heterogeneous group of tumors comprising at least 17 different entities according to the most recent WHO classification (1991). Because of the large number of tumor types and the wide morphological spectrum that these tumors show they constitute a well recognized diagnostic problem. The most frequent chromosome abnormalities so far observed among malignant salivary gland tumors are deletions or translocations involving 6q. Most of the deletions so far encountered are terminal deletions with breakpoints located at 6q22-q25. However, there are also a few cases on record with interstial 6q deletions. The minimal common region lost in the majority of all cases is 6q24-qter. The 6q deletions are not restricted to certain types of malignant salivary gland tumors, but seem to occur in all major histologic subtypes, including adenoid cystic carcinoma, adenocarcinoma, undifferentiated carcinoma, mucoepidermoid carcinoma and acinic cell carcinoma. The frequency of 6q rearrangements vary somewhat in different tumor types. In for example adenoid cystic carcinomas clonal 6q deletions or translocations have been found in nearly 50 % of the cases. The fact that the 6q deletions are often the sole karyotypic change, indicate that they are primary cytogenetic events of pathogenetic importance to malignant salivary gland tumors. In addition to 6q deletions a subgroup of adenoid cystic carcinomas are characterized by a t(6;9)(q21-24;p13-23). This is a primary abnormality that is diagnostic for this tumor types. It should also be noted that in addition to malignant salivary gland tumors there are several other tumor types with recurrent deletions of 6q, including e.g. various types of leukemias and lymphomas, malignant melanomas and neuroblastomas.

### EXAMPLE 9 Involvement of PLAG2 in malignant salivary gland tumors

This example is performed to test whether the different 6q deletions commonly seen in different histologic subtypes of malignant salivary gland tumors involve the PLAG2 gene at 6q24.

### 8.1 Material and methods

FISH analysis using the PLAG2 containing CEPH mega-YACs 798D8 and 921C2 was performed on metaphase chromosomes from two cases of malignant salivary gland tumors, one adenocarcinoma and one adenoid cystic carcinoma, with 6q deletions. The methods for cytogenetic analysis and FISH were the same as described for PLAG1.

### 8.2 Results

Hybridization with both YACs on metaphases from the adenocarcinoma resulted in signals of the same intensity on both chromosome 6 homologs at the expected position. There was no evidence of deletion of PLAG2 signals in this case. In contrast, the adenoid cystic carcinoma contained a subpopulation of cells, which in addition to the signal from the normal chromosome 6 showed either no signal or a weak signal from the 6q-marker chromosome. Both YACs gave a similar hybridization pattern.

### 8.3 Conclusion

Preliminary studies suggest that at least one of the breakpoints in the 6q deletions in malignant salivary gland tumors map in the vicinity of the PLAG2 locus. PLAG2 is therefore a candidate gene for the commonly ocurring 6q deletions in malignant salivary gland tumors.

### EXAMPLE 10

### Animal tumor models involving PLAG1 as tools in in vivo therapeutic drug testing

On the basis of the acquired PLAG1 knowledge, animal tumor models can be developed as tools for in vivo therapeutic drug testing. To achieve this objective (for instance for pleomorphic adenoma of the salivary gland), two approaches can be used, gene transfer (generation of transgenic animals) on the one hand and gene targeting technology (mimicking in vivo of a specific genetic aberration via homologous recombination in embryonic stem cells (ES cells)) on the other. These technologies allow manipulation of the genetic constitution of complex living systems in specific and pre-designed ways. For extensive technical details, see B. Hogan, R. Beddington, F. Constantini, and E. Lacy; In: Manipulating the mouse embryo, a laboratory manual. Cold Spring Harbor Press, 1994; ISBN 0-87969-384-3.

To aim at the mutation of the PLAG1 gene, specifically in selected cell types and selected moments in time, the recently described Cre/LoxP system can be used (Gu, H. et al. Deletion of a DNA polymerase β gene segment in T cells using cell type-specific gene targeting. **Science 265**, 103-106, 1994). The Cre enzyme is a recombinase from bacteriophage P1 whose physiological role is to separate phage genomes that become joined to one another during infection. To achieve so, Cre lines up short sequences of phage DNA, called loxP sites and removes the DNA between them, leaving one loxP site behind. This system has now been shown to be effective in mammalian cells in excising at high efficiency chromosomal DNA. Tissue-specific inactivation or mutation of a gene using this system can be obtained via tissue-specific expression of the Cre enzyme.

As an example, the development of animal model systems for pleomorphic adenoma of the salivary glands using a member of the PLAG gene family will be outlined below, such that the models will be instrumental in in vivo testing of therapeutic drugs.

Two approaches will be followed:
a) in vivo induction of specific genetic aberrations as observed in human patients ((conditional) gene (isogenic) targeting approach); and
b) introduction of DNA constructs representative for the genetic aberrations observed in patients (gene transfer approach).

DNA constructs to be used in gene transfer will be generated on the basis of observations made in patients suffering from pleomorphic adenomas of the salivary glands as far as structure and expression control are concerned; e.g. PLAG1 fusion genes with various translocation partner genes, especially the preferential translocation partner gene CTNNB1 of chromosome 3, i.e, complete PLAG1 under control of a strong (salvary gland-specific) promoter.

### EXAMPLE 11

### The preparation of antibodies against PLAG-encoded proteins

One type of suitable molecules for use in diagnosis and therapy are antibodies directed against the PLAG genes. Two approaches have been followed to develop them. Based upon the nucleotide sequence of the PLAG cDNAs, computer analysis was used to predict the location of antigenic determinants within the proteins. Synthetic peptides containing such determinants were prepared and rabbits were immunized with these. As an alternative approach, cDNA sequences can be expressed in appropriate pro- and eukaryotic expression systems and the polypeptides synthesized in this way can be purified and used for immunization of mice. Cell lines obtained upon transfection or electroporation of constructs of the PLAG genes were helpful in characterizing the antibodies obtained.

For the preparation of rabbit polyclonal antibodies directed against the PLAG1-encoded proteins, use was made of the following three commercially available peptides:
(H-DLSEVRDTQKVPSGKR) 8-Multiple Antigen Peptide
(H-FSSTSYAISIPEKEQPL) 8-MAP
(H-QLPTQTQDLQDP) 8-MAP
obtainable from Research Genetics Inc., Huntsville, AL, USA. The polyclonal antibodies were made according to standard techniques.

With respect to PLAG2, suitable antibodies were generated using hybrid proteins as antigens. These contained various portions of the PLAG2 coding region fused in frame to GST (glutathion-S-transferase).

### EXAMPLE 12

### Inhibition of PLAG1 expression in tumor cells using antisense PLAG1 constructs.

### 1. Introduction

PLAG1 can be used as target in novel therapeutic protocols for tumors in which it is implicated; e.g. pleomorphic adenomas of the salivary glands, uterine leiomyoma, etc. Cancer as a genetic disease is a logical target for gene therapy, either by replacing the missing or inactivated gene or by suppressing the activity of an unwanted gene. The high affinity of short DNA sequences for their target mRNA has indicated that antisense oligonucleotides constitute valuable reagents to specifically suppress the production of gene products, both in vitro and in vivo. Applications have been documented in various biomedical research areas, such as for instance cancer research, virology, and cardiovascular research. The in vitro activity, toxicity, and pharmacokinetic data of antisense oligonucleotides are encouraging and in vivo animal experiments demonstrating suppression of neointimal formation seem very promising. Also in the field of cancer research promising results have been obtained. However, potential nonspecific effects of antisense oligonucleotides should be carefully considered when antisense agents are used to define biological functions of specific genes.

Since expression of the PLAG1 gene is frequently activated or strongly elevated in a wide variety of tumors and tumor cell lines, derived from tumors (rhabdomyosarcoma, uterine leiomyosarcoma, uterine leiomyoma, malignant salivary gland tumors) as well as leukemias and lymphomas, it was speculated that the PLAG1-encoded protein might play a key role in transformation of cells. This example shows that expression of the PLAG1 gene can be strongly reduced by expressing antisense PLAG1 sequences and that reduction of PLAG1 levels in tumor cells results in reversion of the transformed phenotype. Thus the expression or administration of antisense molecules can be successfully applied therapeutically.

### 2. Materials and methods

### 2.1 Tumor cell lines

Tumor cell lines were generated from primary tumors as described by Kazmierczak et al., Genes Chromosom. Cancer **5**:35-39, 1992.

### 2.2 Culturing tumor cells

Tumor cells were propagated in TC199 culture medium with Earle's salts, supplemented with 20% fetal bovine serum (GIBCO), 200 IU/ml penicillin, and 200 microgram/ml streptomycin.

### 2.3 Transfection assay

Transfections were performed using various protocols, namely:
1. The calcium phosphate precipitation procedure of Graham and Van der Eb ("A new technique for the assay of the infectivity of human adenovirus." Virology **52**: 456-467, 1973.).
2. Lipofection: Transfections were carried out using liposome-mediated DNA transfer (lipofectamine, GIBCO/BRL) according to the guidelines of the manufacturer.

### 2.4 Antisense constructs

Sense and antisense constructs of the PLAG1 gene were obtained by inserting human PLAG1 cDNA sequences in both the sense and antisense orientation in expression vectors under transcriptional control of various promoter contexts, e.g. the long terminal repeat of Moloney murine leukemia virus, a CMV promoter, or the early promoter of SV40. For example, the CMV/PLAG1 plasmid was constructed by cloning a human PLAG1 cDNA fragment containing all coding sequences of human PLAG1 in pRC/CMV (Invitrogen) allowing expression under control of the human cytomegalovirus early promoter and enhancer, and selection for G418 resistance.

### 3. Results

### 3.1 Inhibition of PLAG1 expression

Inhibition of PLAG1 expression was observed in tumor cells after induction of antisense PLAG1 expression in these tumor cells. Immunoprecipitation and Western blot analysis indicated a strong reduction of PLAG1 protein levels in the cells expressing antisense PLAG1 sequences. Therefore, this approach can be used therapeutically in tumors with involvement with PLAG1.

### REFERENCES

Albertsen, H.M. et al. (1990). Construction and characterization of a yeast artificial chromosome library containing seven haploid human genome equivalents. Proc. Natl. Acad. Sci. USA **87**: 4256-4260.
Altschul, S.F. et al. (1990). Basic local alignment search tool. J. Mol. Biol. **215**: 403-410.
Blunt, T. et al. (1995). Defective DNA-dependent protein kinase activity is linked to V(D)J recombination and DNA repair defects associated with the murine scid mutation. Cell **80**: 813-823.
Bullerdiek, J. et al. (1993). Cytogenetic subtyping of 220 salivary gland pleomorphic adenomas: correlation to occurrence, histological subtype, and in vitro cellular behavior. Cancer Genet. Cytogenet. **65**: 27-31.
Brunton, L.L., and Lupton, S.D. (1990). An STS in the human IL7 gene located at 8q12-13. Nucl. Acids Res. **18**: 1315.
Chu, G. et al. (1986). Separation of large DNA molecules by contour-clamped homogeneous electric fields. Science **234**: 1582-1585.
Chumakov, I. et al. (1992). Continuum of overlapping clones spanning the entire human chromosome 21q. Nature **359**: 380-387.
Cohen, D. et al. (1993). A first-generation physical map of the human genome. Nature **366**: 698-701.
Cohen, J.C.C. et al. (1992). Cloning of the human cholesterol 7a-hydroxylase gene (CYP7) and localization to chromosome 8q11-q12. Genomics **14**: 153-161.
Corey, S.J., and Shapiro, D.N. (1994). Localization of the human gene for Src-related protein tyrosine kinase LYN to chromosome 8q11-12: a lymphoid signaling cluster? Leukemia **8**: 1914-1917.
Craig, J.M., and Bickmore, W.A. (1993). Chromosome bands-Flavours to savour. Bioessays **15**: 349-354.
Doerflinger, N. et al. (1995). Ataxia with Vitamin E deficiency: refinement of genetic localization and analysis of linkage disequilibrium by using new markers in 14 families. Am. J. Hum. Genet. **56**: 1116-1124.
Feinberg, A.P., and Vogelstein, B. (1984). A technique for radiolabelling DNA restriction endonuclease fragments to high specific activity. Anal. Biochem. **132**: 6-13.
Geurts, J. et al. (1994). Improved procedure for rapid isolation and sequencing of DNA insert termini in yeast artificial chromosomes. Meth. Mol. Cell. Biol. **4**: 257-265.
Green, E.D., and Olson, M.V. (1990). Systematic screening of yeast artificial-chromosome libraries using the polymerase chain reaction. Proc. Natl. Acad. Sci. U.S.A. **87**: 1213-1217.
Gyapay, G. et al. (1994). The 1993-94 Genethon human genetic linkage map. Nature Genetics **7**: 246-339.
Mock, B.A. et al. (1996). Mouse chromosome 4. Mammalian Genome **6**: S73-S96.
Nelson, D.L. et al. (1989). Alu polymerase chain reaction: A method for rapid isolation of human-specific sequences from complex DNA sources. Proc. Natl. Acad. Sci. U.S.A. **86**: 6686-6690.
Petit, M. R. et al. (1996). LPP, the preferred fusion partner gene of HMGIC in lipomas is a novel member of the LIM protein gene family. Genomics **36**: 118-129.
Rychlik, W., and Rhoads, R.E. (1989). A computer program for choosing optimal oligonucleotides for filter hybridization, sequencing and in vitro amplification of DNA. Nucleic Acids Res. **17**: 8543-8551.
Sambrook, J., Fritsch, E.F., and Maniatis, T. (1989). Molecular Cloning: A Laboratory Manual. Cold Spring Harbor Laboratory Press, Cold Spring Harbor, NY.
Sandros, J. et al. (1990). Cytogenetic and molecular observations in human ans experimental salivary gland tumors. Cancer Genet. Cytogenet. **44**: 153-167.
Sapru, M. et al. (1994). A panel of radiation hybrids for chromosome 8. Genomics **21**: 208-216.
Seifert, G. et al. (1986). Diseases of the salivary glands. Pathology Diagnosis. Treatment. Facial nerve surgery. (Translated by P.M. Stell.) Thieme, Stuttgart, New York, pp. 182-194.
Schoenmakers, E.F.P.M. et al. (1994). Physical mapping of chromosome 12q breakpoints in lipoma, pleomorphic salivary gland adenoma, uterine leiomyoma, and myxoid liposarcoma. Genomics **20**: 210-222.
Schoenmakers, E.F.P.M. et al. (1995). Recurrent rearrangements in the high mobility group protein gene, HMGI-C, in benign mesenchymal tumours. Nature Genetics **10**: 436-444.
Testa, J.R. et al. (1988). Localization of the proto-oncogene MOS to 8q11-q12 by in Situ chromosomal hybridization. Genomics **3**: 44-47.
Tomfohrde, J. et al. (1992). Human chromosome 8 linkage map based on short tandem repeat polymorphisms: effect of genotyping errors. Genomics **14**: 144-152.
Van Ommen, G.J. (1993). First report of the HUGO YAC committee. In "Genome Priority Reports" (A.J. Cuticchia, P.L. Parson, and H.P. Klinger, Eds.), Vol. 1, pp 885-888, Karger, Basel.
Wood, S. et al. (1992). Characterization of a human chromosome 8 cosmid library constructed from flow-sorted chromosomes. Cytogenet. Cell Genet. **59**: 243-247.
Yasuda, K. et al. (1994). Localization of the kappa opioid receptor gene to human chromosome band 8q11.2. Genomics **19**: 596-597.
Schoenmakers, E.F.P.M. et al. Recurrent rearrangements in the high mobility group protein gene, HMGIC, in benign mesenchymal tumours. Nature Genetics **10**: 436-444 (1995).
Ashar, H.R. et al. Disruption of the architectural factor HMGI-C: DNA-binding AT hook motifs fused in lipomas to distinct transcriptional regulatory domains. Cell **82**: 57-65 (1995).
Bustin, M. et al. Structural features of the HMG chromosomal proteins and their genes. Biochim. Biophys. Acta **1049**: 231-243 (1990).
Wolffe, A.P. Architectural transcription factors. Science **264**: 1100-1101 (1994).
Schmeichel, K.L. & Beckerle, M.C. The LIM domain is a modular protein-binding interface. Cell **79**: 211-219 (1994).
Seifert, G. et al. WHO international histological classification of tumours: tentative histological classification of salivary gland tumours. Pathol. Res. Pract. **186**: 555-581 (1990).
Mitelman, F. Catalog of Chromosome Aberrations in Cancer. 4th ed., New York, Wiley- Liss (1994).
Mark, J. et al. The mixed salivary gland tumor - a normally benign human neoplasm frequently showing specific chromosome abnormalities. Cancer Genet. Cytogenet. **2**: 231-241 (1980).
Peifer, M. Cancer, catenins, and cuticle pattern: a complex connection. Science **262**: 1667-1668 (1993).
Bellefroid, E.J. et al. The human genome contains hundreds of genes coding for finger proteins of the Kruppel type. DNA **8**: 377-387 (1989).
Sachs, A.B. Messenger RNA degradation in eukaryotes. Cell **74**: 413-421 (1993).
Nollet, F. et al. Genomic organization of the human β-catenin gene (CTNNB1). Genomics **32**: 413-424 (1996).
Kraus, C. et al. Localization of the human β-catenin gene (CTNNB1) to 3p21: a region implicated in tumor development. Genomics **23**: 272-274 (1994).
Gemmill, R.M. et al. A second-generation YAC contig map of human chromosome 3. Nature **377**: 299-319 (1995).
Aplan, P.D. et al. Involvement of the putative hematopoietic transcription factor SCL in T-cell acute lymphoblastic leukemia. Blood **79**: 1327-1333 (1992).
El-Baradi, T. & Pieler, T. Zinc finger proteins: what we know and what we would like to know. Mech. Develop. **35**: 155-169 (1991).
Haber, D.A. et al. An internal deletion within an 11p13 zinc finger gene contributes to the development of Wilms' tumor. Cell **61**: 1257-1269 (1990).
Mitchell, P.J. & Tjian, R. Transcriptional regulation in mammalian cells by sequence-specific DNA binding proteins. Science **245**: 371-378 (1989).
Gumbiner, B.M. & McCrea, P.D. Catenins as mediators of the cytoplasmic functions of cadherins. J. Cell Sci. **17**: 155-158 (1993).
Kemler, R. From cadherins to catenins: cytoplasmic protein interactions and regulation of cell adhesion. Trends Genet. **9**: 317-321 (1993).
Gumbiner, B.M. Cell adhesion: the molecular basis of tissue architecture and morphogenesis. Cell **84**: 345-357 (1996).
Evans, R.W. & Cruickshank, A.H. Epithelial tumours of the salivary glands. Major Probl. Pathol. **1**: 1-299 (1970).
Dardick, I. et al. S-100 protein antibodies do not label salivary gland myoepithelium. Histogenetic implications for salivary gland tumors. Am. J. Pathol. **138**: 619-628 (1991).
Sandros, J. et al. Cytogenetic and molecular observations in human ans experimental salivary gland tumors. Cancer Genet. Cytogenet. **44**: 153-167 (1990).
Green, E.D. & Olson, M.V. Systematic screening of yeast artificial-chromosome libraries using the polymerase chain reaction. Proc. natl. Acad. Sci. U.S.A. **87**: 1213-1217 (1990).
Röijer et al. Identification of a Yeast Artificial Chromosome spanning the 8q12 translocation breakpoint in pleomorphic adenomas with t(3;8)(p21:q12). Genes, Chromosomes & Cancer **16**: 166-171 (1996).
Dib, C. et al. A comprehensive genetic map of the human genome based on 5264 microsatellites. Nature **380**: 152-154 (1996).

## Claims

1. Gene being in an isolated form and implicated in tumorigenesis having the nucleotide sequence of any one of the strands of any one of the members of the PLAG subfamily of zinc finger protein genes or CTNNB1 genes, including modified versions thereof.

2. Tumorigenesis gene (T-gene) as claimed in claim 1 having at least homology with the zinc finger domains that are typical for the PLAG1 gene the nucleotide sequence of which is depicted in figure 4A, or the complementary strand thereof, including modified or elongated versions of both strands.

3. T-gene as claimed in claim 1 or 2 having essentially the nucleotide sequence of the PLAG1 gene as depicted in figure 4A, or the complementary strand thereof, including modified or elongated versions of both strands.

4. T-gene as claimed in claim 1 or 2 having essentially the nucleotide sequence of the PLAG2 gene as depicted in figure 8A, or the complementary strand thereof, including modified or elongated versions of both strands.

5. T-gene as claimed in claim 1 having essentially the nucleotide sequence of the CTNNB1 gene as depicted in figure 9, or the complementary strand thereof, including modified or elongated versions of both strands.

6. T-gene as claimed in claims 1-5 for use as a starting point for designing suitable expression-modulating compounds or techniques for the treatment of non-physiological proliferation phenomena in human or animal.

7. T-gene as claimed in any one of the claims 1-6 for use as a starting point for designing suitable nucleotide probes for (clinically/medically) diagnosing cells having a non-physiological proliferative capacity as compared to wildtype cells.

8. T-gene as claimed in claims 1-7, which gene is implicated in tumors selected from rhabdomyosarcoma, uterine leiomyosarcoma, uterine leiomyoma, malignant salivary gland tumors, leukemias and lymphomas.

9. Protein encoded by a T-gene as claimed in claims 1-8 for use as a starting point for preparing suitable antibodies for (clinically/medically) diagnosing cells having a non-physiological proliferative capacity as compared to wildtype cells.

10. Derivatives of the T-gene as claimed in claim 1-8 for use in diagnosis and the preparation of therapeutical compositions, wherein the derivatives are selected from the group consisting of sense and anti-sense cDNA or fragments thereof, transcripts of the gene or practically usable fragments thereof, fragments of the gene or its complementary strand, proteins encoded by the gene or fragments thereof, antibodies directed to the gene, the cDNA, the transcript, the protein or the fragments thereof, as well as antibody fragments.

11. In situ diagnostic method for diagnosing interphase and/or metaphase cells having a non-physiological proliferative capacity, comprising at least some of the following steps:
a) designing a set of nucleotide probes based on the information obtainable from the nucleotide sequence of the T-gene as claimed in claim 1-5, wherein at least one of the probes is hybridisable to a region of the aberrant gene substantially mapping at the same locus as a corresponding region of the wildtype gene and/or the same or another probe is hybridisable to a region of the aberrant gene mapping at a different locus than a corresponding region of the wildtype gene;
b) incubating one or more interphase or metaphase chromosomes or interphase or metaphase cells having a non-physiological proliferative capacity, with the probe(s) under hybridising conditions; and
c) visualising the hybridisation between the probe(s) and the gene.

12. Method of diagnosing cells having a non-physiological proliferative capacity, comprising at least some of the following steps:
a) taking a biopsy of a tumor to obtain cells to be diagnosed;
b) isolating a suitable T-gene-related macromolecule therefrom;
c) analysing the macromolecule thus obtained by comparison with a wildtype reference molecule preferably from the same individual.

13. Method as claimed in claim 12, comprising at least some of the following steps:
a) taking a biopsy of a tumor to obtain cells to be diagnosed;
b) extracting total RNA thereof;
c) preparing at least one first strand cDNA of the mRNA species in the total RNA extract, which cDNA comprises a suitable tail;
d) performing a PCR and/or RT-PCR using a PLAG gene specific primer and a tail-specific and/or partner-specif/nested primer in order to amplify PLAG gene specific cDNA's;
e) separating the PCR products on a gel to obtain a pattern of bands;
f) evaluating the presence of aberrant bands by comparison to wildtype bands, preferably originating from the same individual.

14. Method as claimed in claim 12, comprising at least some of the following steps:
a) taking a biopsy of a tumor to obtain cells to be diagnosed;
b) isolating total protein therefrom;
c) separating the total protein on a gel to obtain essentially individual bands and optionally trnasferring the bands to a Western blot;
d) hybridising the bands thus obtained with antibodies directed against a part of the protein encoded by the remaining part of the T-gene and against a part of the protein encoded by the substitution part of the T-gene;
e) visualising the antigen-antibody reactions and establishing the presence of aberrant bands by comparison with bands from wildtype proteins, preferably originating from the same individual.

15. Method as claimed in claim 12, comprising at least some of the following steps:
a) taking a biopsy of a tumor to obtain cells to be diagnosed;
b) isolating total DNA therefrom;
c) digesting the DNA with one or more so-called "rare cutter" restriction enzymes;
d) separating the digest thus prepared on a gel to obtain a separation pattern;
e) optionally transfering the separation pattern to a Southern blot;
f) hybridising the separation pattern in the gel or on the blot with one or more informative probes under hybridising conditions;
g) visualising the hybridisations and establishing the presence of aberrant bands by comparison to wildtype bands, preferably originating from the same individual.

16. Method as claimed in any one of the claims 10-15, wherein the cells having a non-physiological proliferative capacity are selected from the group consisting of the tumors pleomorphic adenomas of the salivary gland, lipoblastomas, uterine leiomyomas, and other benign tumors as well as various malignant tumors, including but not limited to sarcomas (e.g. rhabdomyosarcoma, malignant salivary gland tumors) and leukemias and lymphomas.

17. Anti-sense molecules of a T-gene as claimed in claims 1-8 for use in the treatment of diseases involving cells having a non-physiological proliferative capacity by modulating the expression of the gene.

18. Expression inhibitors of the T-gene as claimed in claims 1-8 for use in the treatment of diseases involving cells having a non-physiological proliferative capacity.

19. Diagnostic kit for performing the method as claimed in claim 11, comprising a suitable set of labeled nucleotide probes.

20. Diagnostic kit for performing the method as claimed in claim 12, comprising a suitable set of labeled probes.

21. Diagnostic kit for performing the method as claimed in claim 13, comprising a suitable set of labeled T-gene specific and tail specific PCR primers.

22. Diagnostic kit for performing the method as claimed in claim 15, comprising a suitable set of labeled probes, and suitable rare cutting restriction enzymes.

23. Method for isolating other T-gene based on the existence of a fusion gene, fusion transcript or fusion protein in a tumor cell by using at least a part of a T-gene for designing molecular tools (probes, primers etc.).

24. T-gene obtainable by the method of claim 23.

25. T-gene as claimed in claim 24 for use in diagnostic or therapeutic methods.


SEQUENCE LISTING

(1) GENERAL INFORMATION:

(i) APPLICANT:
(A) NAME: K.U. Leuven Research & Development
(B) STREET: Groot Begijnhof, Benedenstraat 59
(C) CITY: Leuven
(E) COUNTRY: Belgium
(F) POSTAL CODE (ZIP): 3000
(G) TELEPHONE: --
(H) TELEFAX: --
(I) TELEX: --

(ii) TITLE OF INVENTION: Plag gene family and tumorigenesis

(iii) NUMBER OF SEQUENCES: 5

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 97200130.9
(vi) PRIOR APPLICATION DATA:
(A) APPLICATION NUMBER: EP 96202339.6
(B) FILING DATE: 22-AUG-1996

(2) INFORMATION FOR SEQ ID NO: 1:

(i) SEQUENCE CHARACTERISTICS:
(A) LENGTH: 7313 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(ix) FEATURE:
(A) NAME/KEY: CDS
(B) LOCATION:481..1983

(xi) SEQUENCE DESCRIPTION: SEQ ID NO: 1:

GGCAGCGCAT ACACTACAAT GGCTGCTGGA AAGAGGCGTA AGGAAACAAT TTCCAGGCCC 60

GCCGCGTCCA GCCCGAAATA TGAGAAAAAA ATTATTAGAA ATTCCGCGGG CGGTGTAGAG 120

GCGGCGGACG GGCCGGAGGG AGGATGTTAA AGCCCCGCGG TTGCCTCTTG GTGCTGCCTT 180
GGCCGTATTT GGCACCCAGA ATGCTTCATT CTGTGACGGT CTATTAATAA GGTTGCCTTG 240
CTAGAGTTTG GAGCAGGGCC TCAGATTGGC CAAAATGGGA AGGATTGGAT TCCACTCTCT 300
TCCACGAAGA GTCAATGGGA CTGGCTAAGA TCAAAGTCTG AGGCTTTTTC CATCAGTAAT 360
CAGTCCCTTT TTGCTTTCTT TTACGACCAC ATGAAACTTG AGAAGCCACC TAAAGCTATA 420
TCATTTAGTG GAGTTGGGCA GTTCCCAAGT GTCCAACAAG AAGGCCTGGT TTAGGCTGCG 480
ATGGCCACTG TCATTCCTGG TGATTTGTCA GAAGTAAGAG ATACCCAGAA AGTCCCTTCA 540
GGGAAACGTA AGCGTGGTGA AACCAAACCA AGAAAAAACT TTCCTTGCCA ACTGTGTGAC 600
AAGGCCTTTA ACAGTGTTGA GAAATTAAAG GTTCACTCCT ACTCTCACAC AGGAGAGAGG 660
CCCTACAAGT GCATACAACA AGACTGCACC AAGGCCTTTG TTTCTAAGTA CAAATTACAA 720
AGGCACATGG CTACTCATTC TCCTGAGAAA ACCCACAAGT GTAATTATTG TGAGAAAATG 780
TTTCACCGGA AAGATCATCT GAAGAATCAC CTCCATACAC ACGACCCTAA CAAAGAGACG 840
TTTAAGTGCG AAGAATGTGG CAAGAACTAC AATACCAAGC TTGGATTTAA ACGTCACTTG 900
GCCTTGCATG CCGCAACAAG TGGTGACCTC ACCTGTAAGG TATGTTTGCA AACTTTTGAA 960
AGCACGGGAG TGCTTCTGGA GCACCTTAAA TCTCATGCAG GCAAGTCGTC TGGTGGGGTT 1020
AAAGAAAAAA AGCACCAGTG CGAACATTGT GATCGCCGGT TCTACACCCG AAAGGATGTC 1080
CGGAGACACA TGGTGGTGCA CACTGGAAGA AAGGACTTCC TCTGTCAGTA TTGTGCACAG 1140
AGATTTGGGC GAAAGGATCA CCTGACTCGA CATATGAAGA AGAGTCACAA TCAAGAGCTT 1200
CTGAAGGTCA AAACAGAACC AGTGGATTTC CTTGACCCAT TTACCTGCAA TGTGTCTGTG 1260
CCTATAAAAG ACGAGCTCCT TCCGGTGATG TCCTTACCTT CCAGTGAACT GTTATCAAAG 1320
CCATTCACAA ACACTTTGCA GTTAAACCTC TACAACACTC CATTTCAGTC CATGCAGAGC 1380
TCGGGATCTG CCCACCAAAT GATCACAACT TTACCTTTGG GAATGACATG CCCAATAGAT 1440
ATGGACACTG TTCATCCCTC TCACCACCTT TCTTTCAAAT ATCCGTTCAG TTCTACCTCA 1500
TATGCAATTT CTATTCCTGA AAAAGAACAG CCATTAAAGG GGGAAATTGA GAGTTACCTG 1560
ATGGAGTTAC AAGGTGGCGT GCCCTCTTCA TCCCAAGATT CTCAAGCATC GTCATCATCT 1620
AAGCTAGGGT TGGATCCTCA GATTGGGTCC CTAGATGATG GTGCAGGAGA CCTCTCCCTA 1680
TCCAAAAGCT CTATCTCCAT CAGTGACCCC CTAAACACAC CAGCATTGGA TTTTTCTCAG 1740
TTGTTTAATT TCATACCTTT AAATGGTCCT CCCTATAATC CTCTATCAGT GGGGAGCCTT 1800

GGAATGAGCT ATTCCCAGGA AGAAGCACAT TCTTCTGTTT CCCAGCTCCC CACACAAACA 1860
CAGGATCTTC AGGATCCTGC AAACACTATA GGGCTTGGGT CTCTGCACTC ACTGTCAGCA 1920
GCTTTCACCA GCAGTTTAAG CACAAGTACC ACCCTCCCAC GTTTCCATCA AGCTTTTCAG 1980
TAGGATTCTG GGACATGGAT TCATTACAGA AATGTATGTG TAGCTGTGCC CTAGATGACC 2040
ATTTTTATTT TAGTGCCTAC TTTAAAACAG TATAAAAATT TCTGCTTTTG TATAATACAA 2100
ATTTTCATTA AGCCAGTATA AAATAGAAAC TAGCTTTTAA ACTGAGCTTT GGAACCATTT 2160
GTGTTCAGTT AAGTTTACCT GGGTATTTTG TCCTGATTCA CTGCCAATTG TCACATTTTA 2220
AGACTTTTTT TTTTTCCATA TAGGAAAGCC ATTATTAGTA GTAAACTTTT ACAAATCCCA 2280
TTTTCAAATT ACTTTTAGAT CTTAAAATTT TCATTTTTGT CTAATAACAG TGGCTCTACC 2340
TTTTGACATC TGGCTCATTA AAAAATTTAG CAATAGAATG TAAATTGTAT AAAAAGTTTG 2400
TGAATAACTC AAGGGTTTAA ATTTTCTTAC TAGCTTCTAA ATGGATTAAT AATCAAGTGC 2460
TTCAAATGAA TTAAGAGTCC AGTTTCGGAA GATAATAAAT GTTTGTTAGA TACACCATAA 2520
TTTCAGATCA GTATATTCTG AAGACTCTCT GTTGTCTGGC TAAAATATTT GCCATCTTTA 2580
TTATGAGCCT TTAAGGAAAA CAAACCCTAA ACACAAAGCA TCAGTATTTA TAGCAAAAAG 2640
AGACTCTGTT AGGTGACATG GCATTTCGTG TCACTTAATA GTTGGCCCTA AATTAGTACA 2700
CAGGATATTT TGTCGTGTTT CATCCTTCTT AACATGCTAT CTTTTCATTT AATAATAGTA 2760
ATAGTGTATG GCATTGGGGT CTTCAGAGTC GATATATAGG TAGATCTCTT TAGTCTTTTC 2820
CACCTTTCAC ATCCAAGGGG TGGGTCAAGT GCAGCCAGCA ATTTATTTTC ATTGTTGGCC 2880
CACGGTTAGT CCATAATCTA GAGCCATTGT GGAACTGCAG CCATGAGGTG TGTTTATCCC 2940
ACAGTGGATT GACTCAGCCT CTGTGGGTGA CAGACTTCTA AGCAGGAAGA TAGACGTGAA 3000
GCACATGGTT ACATTTGGGA ACTTGTGTAG GGATCATGGC CCCTGTAGCC AGGGTTAAAA 3060
ACTGGACTTT TTAGAAGTAA AGTAAAAGCA TAKCGCTTAT ATCATTTCTT GCTGAATTTG 3120
ATATGTTTTT CTTTCCCTTA AGAATCAAAA GCAGAAAACA AAAACAACAG TCCTACTCCG 3180
ATGTTATCTT TCTGATTCAA TGTGAATCCA TCTTTCCTTG CAATATTTTG GATGGAGAAT 3240
TTGAAGTTAA ATGCATTAGA AAACTACCTG ATGAACTACC ACAAAGTTTT AAGTGACTAG 3300
AAATATATAC AGTAAAATCC CACTTTCATG CATCTCTGGG AAATGATAGG AGTATTGCAA 3360
ATAAGTTGAG TTTGTAGAGG GTAACAAAGT AAAGTAAAAC AAACCTATCT TGGTTAACAT 3420
GAAAATAACA ATTGAGAATA TATTATATTC ACTGAATAAT TATAGGCTTT TCCTCACATT 3480

AGACAACCAA CATAATCTTC TTAAAGGTCT AATTAATATA TTTTTCTAAG GGTCAGTTGG 3540
GACATTAACC TAAGAAACAT ATCTATTAAG CACTTGTTAA CACCTTATTT TAGGACCCTT 3600
TCCGTTGGGG ATGGGGGCAA GGGTGGGAGG TTTTTAGAAG AGTATATATC TCTTTAAAAA 3660
AAAACAGAAA GAAAAATATT TCTGAGCACT CATTAGCCCT ATATGGAAAC TTCTTTCCTT 3720
TTTGTAGGGC CAGTTATCAC TGCAGATTGC AATGTTTACC AAGAATTTCT AAAAATGAGT 3780
GCAGATTACT GAATATAATA CATTATTTAA AATATTTGGG AGTAGTATAA TTTGTTGAGA 3840
AATGTAAATT GTAATAATGT AAATGGGGGG CTTCAATATA TATATATAAT ACACACACAC 3900
ACACACATGC ACACATACCG CACTTCATAG AATCAAAGTT GCTCTCTGAA GGAGCTTTGG 3960
CTCCTGATAT TTTATCATGC TCCTATATTT TTTTAATCCT TGGAGCAGTA GTTTTTATAC 4020
TTATGTATTT AAATTTTATT ATGAAAAATT ACATTTATTA AAAAAGTGTG TTCCAAAGGC 4080
ATTAAAATTA TATATGTTAA TAAGGAAGTA CATTTTTAAA TTTTTCAAAC TGCTCCTAGC 4140
TTTTGATTAG GAGAATATTT TTTCTGAAAG TAGGCTTTTC GCTCTGCTTC ATTACTGCTT 4200
CCTTTAGTTT CTATGAAACA GATTGCTTAC CTAAATCTTT AGTTGAATGA TTAGTGTTCA 4260
ATATTGCTTT AATCACCATA TAAAAGGAAA AAAATTGGTG ACAGAGCACA AATAGAAAAC 4320
CTATTTTTAA ATAGAAATCA CAAATAGCAA GTGTGGAAGC ACTACTTTAT TCTGTTTAAA 4380
ATGTACTTAA GAAGTCATCA AATTAGTGAA CTGAGACATT GGCCTTAGTA GGCTGTATTC 4440
ACTGCTAATT TAAAAAAGGG AGTACCAGGA TTTATTAAGT AAAGCATTTT GGAAATGGGG 4500
AATAGCGCCA TATATGTATG TATGTGTATG TGTGTGTGTG GTGTGTGTAT ATATACACAC 4560
ACACATACAT ACTTAAATCT TGCCCTGCAT GAAATTCAAA TACATGGAGG CACATCTTCA 4620
GGGCACCAGT GTTAAAATTT TGGAGTCTTA ATTTTCATGT GTACACCTCT TTGCCTGTTC 4680
CCACCCCCAG ACTTGAAATA ACACTTCAGA GTAAGAGGGA ATTCAGCTAA TTTGTTTTTA 4740
AAATTGACTG TAGTGGTCAC TAAACCCTTT TTGAGAGAAT TTCTATTAAA GATGAGGCAG 4800
ACTCGCTTAT TTGAATTGCA CAATGTTCTA ACAAGGATGT AACACAGAAT TGGCTTTTTT 4860
TTCCCTAGAA AAAGATTGTT TGTTTCTATG TCAACTAGAT ATGATTAAAA ATAAGTATTG 4920
CCAATGCTGT TTTCATTCTC TAGTGGCCAG AATCATTATC CTTGAAATTT CTGGTAGTGC 4980
CTTAGCTTGG TTAAAAAAAA AAAAAAAAAA AAAAAAAAAG GGATTAACAT TAAATAAAAG 5040
TAGTTTAGAA TTTGGGCCTC AGACAAGATA TTGAACCTCA TTCAGTTTCA CTTCCACATG 5100
TATGTACAAG TTAGGTCACC AAACACGGAA GTTGAGTGTG GAAGGATCTT GGCACTGTAA 5160

GCAATGCTAT CCATTGATGT ATACAAGTAC CTTTATAGTT ATCGATCACT GTTAAAACTT 5220
TCATTTTAAA ATCCTATTAC CAAGTTCAGT TTTTTAAAAC TTCAATTGTC CTGGCTGATT 5280
ATGCATCACT CTGTGTGCAA CTTTTTTATT TCATTTAGTG TTTCTTTCAA GCTGTGTATT 5340
TTTGCCTATT TGTTGCTTGT GCTTTATTTT TCTTAGTCAT TTGTGGAATA TAGTGATATA 5400
TTGTGTTAAT TTGGACAGTA GCGGTTTTTA AAAACCATAT ACTGACTGAA ACATGAGCCA 5460
GAGCCGATTG CTTTATTAAG CTAATAATGA ATGTTAAAGA GTACATATTT TCAGGATCGT 5520
TCATCTAGTG AGCAATACAC ATATTATAGG CCAATATTTT TTTAAAAAAT AGAGCTTGGT 5580
CAACCTCTAT ACTACACATA TTACAAGATA TAGCACTTTC AAAATGAATC TAAACCTTTA 5640
CAGAAACTTT CTTATAGGTT ATGCCTTTTA TTTTAAGACT TATTATAATT CAAGTGCCAT 5700
TAGATGATAT ATATGTAGGC CTTTGATATA TAATGCTTTG TGTACAAAAA TGGTAGATGG 5760
TATTTTAAAC AGGTACATTT TTACAGTGTT TTCTTATCAA TTTGCTATAT TGCACAGAAT 5820
CAGTGTGTGT CTTTTCATAA GGTTTTACAA TGGTTTATTT TTTTACAAGG TTTACGTGTC 5880
TCAAAGCACA CTGTCTTCCC AGTACGTAAG TTAAAAAATA CCAGTTCACC CAAGTTGCTT 5940
CTAGCCTACT GAGATCCATG TGACATTGGA GGAGATCTTT TAAATGTTTA GTATTCGTCA 6000
TTAGCAATGG CTGGCTGTTA GTTCTGGTAA ATGTGTGCCT AAGTTGAATT TGTCTTGTTT 6060
TTCTCACACT GTGTCAGCAG CCATGTCTAC AACACAGATA AGTCTGTTGT GATCACATAG 6120
ATCTACATAA GTTGTGCAGT TTTGTGCTAA AAACCCATAG GGAGCTCCTT TGGGATCATA 6180
GAAAAGAAGA TCATGCAACC AGCATTGGTG AAGGCACACT CAGATTGCAC TTAGGGCCTT 6240
TCTATGATGT TGTCAACCCT CTGAGGATGG AAGGCAGTGT CTTTTGATGT TATCTAGCCT 6300
AGAAATGACA CAGAACTATT GCTAATGTAT AAAACACTTC ATTATATAAG CTTCAGTGGT 6360
ACAGATGAAC CAGAATGAAT GTTTATCTTC TCAGAAACAC TCCTTCAATA TTATATTGGA 6420
TCATGCTGCT AATGTAACTT GGGCTACAAC TCTTCATGGT GCTACAAACT TCTCTGTCTC 6480
ATTCAGTCGT ATTTTTTTAT CCATAGAAAA AGGACTACAT TAGGTGTAAA AGTGTACAAT 6540
ATATTTTTAT ACTGTGACTT AATTTGTCAT TAACAAACTT TTACACCACC ACAATGTATT 6600
CATGTGCACT TGCAAAAGGA GATCTCGGAC ATGCAAATGT TACCAGAACA AACCCAGCTT 6660
TTGTCCACAA GGTGACTGTA ACTCAGAATG GAAAGTGGGC TTTATAATAG GGTGTGGAGT 6720
GAAGAACATG CTGTATGTTA CTAACAGCCC TTTGAATTTA ACAAAAACTG GGAATCCATT 6780
AGGAAACGGA TTGCATCATA CCTGAACATA AGCTGGACTG CTGAAATTGT ATTTTTAGCT 6840

AATGAAAAAG TGTTTGGACT AGTACTCTAA AAATGTTCTA ATGATAAAGT TTTGAGTCAA 6900

AATAGAAAAG AAAAAAATCT GCATTCCAGG CCGAATTTTG TATATTTTTA TTGCATTTAA 6960

AATTGCTATT CTGTAATATT GGGAAATCAA GTGGCTTATC ATGTATATCG TGTACTTAAA 7020

ATGTATTCAC AAACTACTGT TGTATTTGTA TAAAATATAG ACAAAGATCA TATTTTTTGT 7080

GTGTGTATAA GCTCTGTAAA ATAGCAATCA CATTATGAAG CTGCAGTGAT ACTACATTTT 7140

AAACATTCAC ATCCAAAGAA GCAGACTATT TATTGTCCAT ATACCAGATT TAAAATATTA 7200

ATTTGCTGCT AATTAAATAA TAGTACTGCA GCTTCTTGTG GCCTACAGTG TTATGTTTGC 7260

TGTAAGAATA AGATATGTGA ATTCCACAAA ATATATGAAT AAAATCTCGT GCC 7313

(2) INFORMATION FOR SEQ ID NO: 2:

(i) SEQUENCE CHARACTERISTICS:
(A) LENGTH: 500 amino acids
(B) TYPE: amino acid
(C) STRANDEDNESS:
(D) TOPOLOGY: unknown

(ii) MOLECULE TYPE: protein

(vi) ORIGINAL SOURCE:
(A) ORGANISM: Homo sapiens

(xi) SEQUENCE DESCRIPTION: SEQ ID NO: 2:

Met Ala Thr Val Ile Pro Gly Asp Leu Ser Glu Val Arg Asp Thr Gln
1               5                   10                  15

Lys Val Pro Ser Gly Lys Arg Lys Arg Gly Glu Thr Lys Pro Arg Lys
            20                  25                  30

Asn Phe Pro Cys Gln Leu Cys Asp Lys Ala Phe Asn Ser Val Glu Lys
        35                  40                  45

Leu Lys Val His Ser Tyr Ser His Thr Gly Glu Arg Pro Tyr Lys Cys
    50                  55                  60

Ile Gln Gln Asp Cys Thr Lys Ala Phe Val Ser Lys Tyr Lys Leu Gln
65                  70                  75                  80

Arg His Met Ala Thr His Ser Pro Glu Lys Thr His Lys Cys Asn Tyr
                85                  90                  95

Cys Glu Lys Met Phe His Arg Lys Asp His Leu Lys Asn His Leu His
            100                 105                 110

Thr His Asp Pro Asn Lys Glu Thr Phe Lys Cys Glu Glu Cys Gly Lys
        115                 120                 125

Asn Tyr Asn Thr Lys Leu Gly Phe Lys Arg His Leu Ala Leu His Ala
    130                 135                 140

Ala Thr Ser Gly Asp Leu Thr Cys Lys Val Cys Leu Gln Thr Phe Glu
145                 150                 155                 160

Ser Thr Gly Val Leu Leu Glu His Leu Lys Ser His Ala Gly Lys Ser
                165                 170                 175

Ser Gly Gly Val Lys Glu Lys Lys His Gln Cys Glu His Cys Asp Arg
            180                 185                 190

Arg Phe Tyr Thr Arg Lys Asp Val Arg Arg His Met Val Val His Thr
        195                 200                 205

Gly Arg Lys Asp Phe Leu Cys Gln Tyr Cys Ala Gln Arg Phe Gly Arg
    210                 215                 220

Lys Asp His Leu Thr Arg His Met Lys Lys Ser His Asn Gln Glu Leu
225                 230                 235                 240

Leu Lys Val Lys Thr Glu Pro Val Asp Phe Leu Asp Pro Phe Thr Cys
                245                 250                 255

Asn Val Ser Val Pro Ile Lys Asp Glu Leu Leu Pro Val Met Ser Leu
            260                 265                 270

Pro Ser Ser Glu Leu Leu Ser Lys Pro Phe Thr Asn Thr Leu Gln Leu
        275                 280                 285

Asn Leu Tyr Asn Thr Pro Phe Gln Ser Met Gln Ser Ser Gly Ser Ala
    290                 295                 300

His Gln Met Ile Thr Thr Leu Pro Leu Gly Met Thr Cys Pro Ile Asp
305                 310                 315                 320

Met Asp Thr Val His Pro Ser His His Leu Ser Phe Lys Tyr Pro Phe
                325                 330                 335

Ser Ser Thr Ser Tyr Ala Ile Ser Ile Pro Glu Lys Glu Gln Pro Leu
            340                 345                 350

Lys Gly Glu Ile Glu Ser Tyr Leu Met Glu Leu Gln Gly Gly Val Pro
        355                 360                 365

Ser Ser Ser Gln Asp Ser Gln Ala Ser Ser Ser Ser Lys Leu Gly Leu
    370                 375                 380

Asp Pro Gln Ile Gly Ser Leu Asp Asp Gly Ala Gly Asp Leu Ser Leu
385                 390                 395                 400

Ser Lys Ser Ser Ile Ser Ile Ser Asp Pro Leu Asn Thr Pro Ala Leu
                405                 410                 415

Asp Phe Ser Gln Leu Phe Asn Phe Ile Pro Leu Asn Gly Pro Pro Tyr

420                 425                 430

Asn Pro Leu Ser Val Gly Ser Leu Gly Met Ser Tyr Ser Gln Glu Glu
        435                 440                 445

Ala His Ser Ser Val Ser Gln Leu Pro Thr Gln Thr Gln Asp Leu Gln
    450                 455                 460

Asp Pro Ala Asn Thr Ile Gly Leu Gly Ser Leu His Ser Leu Ser Ala
465                 470                 475                 480

Ala Phe Thr Ser Ser Leu Ser Thr Ser Thr Thr Leu Pro Arg Phe His
                485                 490                 495

Gln Ala Phe Gln
            500

(2) INFORMATION FOR SEQ ID NO: 3:

(i) SEQUENCE CHARACTERISTICS:
(A) LENGTH: 2561 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(ix) FEATURE:
(A) NAME/KEY: CDS
(B) LOCATION:177..1412

(xi) SEQUENCE DESCRIPTION: SEQ ID NO: 3:

AGGCTCAAGA TAAAGACCTT AAAGATAACT TTGTGTGTCT CTCCCTTTCT AGGTATTTGC      60

ATAGGAATCA GAGGAGTTAA TCTTGTCTCT TCTCACAGGT TTGAATCTTC AGACAAACTT     120

CTGGGAGGAC TCGGTCCCTG CCTCGCAGCA GATGTTCCCT GTCACTCAGT AGGCATATGG     180

CTACCCATTC TCCCCAGAAA TCTCACCAGT GTGCTCACTG TGAGAAGACG TTCAACCGGA     240

AAGACCACCT GAAAAACCAC CTCCAGACCC ACGACCCCAA CAAAATGGCC TTTGGGTGTG     300

AGGAGTGTGG GAAGAAGTAC AACACCATGC TGGGCTATAA GAGGCACCTG GCCCTCCATG     360

CGGCCAGCAG TGGGGACCTC ACCTGTGGGG TCTGTGCCCT GGAGCTAGGG AGCACCGAGG     420

TGCTACTGGA CCACCTCAAA GCCCATGCGG AAGAGAAGCC CCCTAGCGGA ACCAAGGAAA     480

AGAAGCACCA GTGCGACCAC TGTGAAAGAT GCTTCTACAC CCGGAAGGAT GTGCGACGCC     540

ACCTGGTGGT CCACACAGGA TGCAAGGACT TCCTGTGCCA GTTCTGTGCC CAGAGATTTG 600
GGCGCAAGGT TCACCTCACC CGGCATACCA AGAAGACCCA CTCACAGGAG CTGATGAAAG 660
AGAGCTTGCA GACCGGAGAC CTTCTGAGCA CCTTCCACAC CATCTCGCCT TCATTCCAAC 720
TGAAGGCTGC TGCCTTGCCT CCTTTCCCTT TAGGAGCTTC TGCCCAGAAC GGGCTTGCAA 780
GTAGCTTGCC AGCTGAGGTC CATAGCCTCA CCCTCAGTCC CCCAGAACAA GCCGCCCAGC 840
CTATGCAGCC GCTGCCAGAG TCCCTGGCCT CCCTCCACCC CTCGGTATCC CCTGGCTCTC 900
CTCCGCCACC CCTTCCCAAT CACAAGTACA ACACCACTTC TACCTCATAC TCCCCACTTG 960
CAAGCCTGCC CCTCAAAGCA GATACTAAAG GTTTTTGCAA TATCAGTTTG TTTGAGGACT 1020
TGCCTCTGCA AGAGCCTCAG TCACCTCAAA AGCTCAACCC AGGTTTTGAT CTGGCTAAGG 1080
GAAATGCTGG TAAAGTAAAC CTGCCCAAGG AGCTGCCTGC AGATGCTGTG AACCTAACAA 1140
TACCTGCCTC TCTGGACCTG TCCCCCCTGT TGGGCTTCTG GCAGCTGCCC CCTCCTGCTA 1200
CCCAAAATAC CTTTGGGAAT AGCACTCTTG CCCTGGGGCC TGGGGAATCT TTGCCCCACA 1260
GGTTAAGCTG TCTGGGGCAG CAGCAGCAAG AACCCCCACT TGCCATGGGC ACTGTGAGCC 1320
TGGGCCAGCT CCCCCTGCCC CCCATCCCTC ATGTGTTCTC AGCTGGCACT GGCTCTGCCA 1380
TCCTGCCTCA TTTCCATCAT GCATTCAGAT AATTGATTTT TAAAGTGTAT TTTTCCGTAT 1440
TCTGGAAGAT GTTTTAAGAA GCATTTTAAA TGTCAGTTAC AATATGAGAA AGATTTGGAA 1500
AACGAGACTG GGACTATGGC TTATTCAGTG ATGACTGGCT TGAGATGATA AGAGAATTCT 1560
CGAACTGCAT GTATTGTGCC AATCTGTCCT GAGTGTTCAT GCTTTGTACC AAATTTAATG 1620
AACGCGTGTT CTGTAATCCA AACTGCAAAT ATTGTCATAA CCAACATCCA AAATGACGGC 1680
TGCTATATAT AAGTGTTTGT CCATATGGGA ATTTAATCCG TAAGCCCATG GATCCATAAT 1740
GTTAAACTAA ATAACTTTAT GGTGGCACTG CCTAGTAAGG GAACTATGGA AAGGTTTGGA 1800
TTTCTCCAAA TCTGGGAGAA TTTTCAAAAT AAGAAAATAA CCTTTATATG ATATACTATG 1860
ACTAGGCTGT GTATTTCTTT TCAGGGATTT TTCTACCTTC AGGGTTGGAT GTAGTTTAGT 1920
TACTATTACC ATAGCCAACC TGTAGTTTTA CATATACATT TTCTTGTGGA GCAATAGAGT 1980
TCTCCATTTT ACAGAAGCAT TTTAAATGTA GTTTGAATAT TTTCCACAAG ATGCTGCAAT 2040
GTGAGTTATC ACTTCATTTA TCTTAAAGAA AGACTAAACT GGTTGTCAGT TACATCTGAC 2100
AGAAAAAAAA AAAAAATCAC TGTGTAACCA GGTTAAGTGG TAAAATAATC CAGGGCGTCA 2160
GTCAAAGGCA TTTTGCTGAC TTTAATATTG ATTATATTTT TAACAGGGAA TTTAAGGAAA 2220

ATATTACCTG GGAATTAAAA AATATATATA TATTAAAACA AGAATTTTCC TTTGCCTCTG 2280

TCTAGCTTAA ACCTACTACC TCAAGCTGCT TAAGKTCCTT AAGTATTGTT TGTAATCACC 2340

AATAAATAAG TGCATTTGTA ATTCATCAGT CATTATTAGC TTTTATTAAA AGAAGATTAC 2400

GTTTTACAAT GTAACTATAA TCTCTTGAAT TTGGTATCTT ATTAATGAGT TTTAAAGATG 2460

TAAAACCTAA CCTTTTTTAA AGCTCCATTG TCTTATGTTT TTAGAGGCTT TTCCGTAAAC 2520

ATATATCTTA CATATAATAA ACTTTTCAAA TCTTGCAAAT T 2561

(2) INFORMATION FOR SEQ ID NO: 4:

(i) SEQUENCE CHARACTERISTICS:
(A) LENGTH: 411 amino acids
(B) TYPE: amino acid
(C) STRANDEDNESS:
(D) TOPOLOGY: unknown

(ii) MOLECULE TYPE: protein

(vi) ORIGINAL SOURCE:
(A) ORGANISM: Homo sapiens

(xi) SEQUENCE DESCRIPTION: SEQ ID NO: 4:

(2) INFORMATION FOR SEQ ID NO: 5:

(i) SEQUENCE CHARACTERISTICS:
(A) LENGTH: 3362 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO: 5:

AAGCCTCTCG GTCTGTGGCA GCAGCGTTGG CCCGGCCCCG GGAGCGGAGA GCGAGGGGAG 60

GCGGAGACGG AGGAAGGTCT GAGGAGCAGC TTCAGTCCCC GCCGAGCCGC CACCGCAGGT 120

CGAGGACGGT CGGACTCCCG CGGCGGGAGG AGCCTGTTCC CCTGAGGGTA TTTGAAGTAT 180

ACCATACAAC TGTTTTGAAA ATCCAGCGTG GACAATGGCT ACTCAAGCTG ATTTGATGGA 240

GTTGGACATG GCCATGGAAC CAGACAGAAA AGCGGCTGTT AGTCACTGGC AGCAACAGTC 300

TTACCTGGAC TCTGGAATCC ATTCTGGTGC CACTACCACA GCTCCTTCTC TGAGTGGTAA 360

AGGCAATCCT GAGGAAGAGG ATGTGGATAC CTCCCAAGTC CTGTATGAGT GGGAACAGGG 420

ATTTTCTCAG TCCTTCACTC AAGAACAAGT AGCTGATATT GATGGACAGT ATGCAATGAC 480

TCGAGCTCAG AGGGTACGAG CTGCTATGTT CCCTGAGACA TTAGATGAGG GCATGCAGAT 540

CCCATCTACA CAGTTTGATG CTGCTCATCC CACTAATGTC CAGCGTTTGG CTGAACCATC 600

ACAGATGCTG AAACATGCAG TTGTAAACTT GATTAACTAT CAAGATGATG CAGAACTTGC 660

CACACGTGCA ATCCCTGAAC TGACAAAACT GCTAAATGAC GAGGACCAGG TGGTGGTTAA 720

TAAGGCTGCA GTTATGGTCC ATCAGCTTTC TAAAAAGGAA GCTTCCAGAC ACGCTATCAT 780

GCGTTCTCCT CAGATGGTGT CTGCTATTGT ACGTACCATG CAGAATACAA ATGATGTAGA 840

AACAGCTCGT TGTACCGCTG GGACCTTGCA TAACCTTTCC CATCATCGTG AGGGCTTACT 900

GGCCATCTTT AAGTCTGGAG GCATTCCTGC CCTGGTGAAA ATGCTTGGTT CACCAGTGGA 960

TTCTGTGTTG TTTTATGCCA TTACAACTCT CCACAACCTT TTATTACATC AAGAAGGAGC 1020

TAAAATGGCA GTGCGTTTAG CTGGTGGGCT GCAGAAAATG GTTGCCTTGC TCAACAAAAC 1080

AAATGTTAAA TTCTTGGCTA TTACGACAGA CTGCCTTCAA ATTTTAGCTT ATGGCAACCA 1140

AGAAAGCAAG CTCATCATAC TGGCTAGTGG TGGACCCCAA GCTTTAGTAA ATATAATGAG 1200

GACCTATACT TACGAAAAAC TACTGTGGAC CACAAGCAGA GTGCTGAAGG TGCTATCTGT 1260
CTGCTCTAGT AATAAGCCGG CTATTGTAGA AGCTGGTGGA ATGCAAGCTT TAGGACTTCA 1320
CCTGACAGAT CCAAGTCAAC GTCTTGTTCA GAACTGTCTT TGGACTCTCA GGAATCTTTC 1380
AGATGCTGCA ACTAAACAGG AAGGGATGGA AGGTCTCCTT GGGACTCTTG TTCAGCTTCT 1440
GGGTTCAGAT GATATAAATG TGGTCACCTG TGCAGCTGGA ATTCTTTCTA ACCTCACTTG 1500
CAATAATTAT AAGAACAAGA TGATGGTCTG CCAAGTGGGT GGTATAGAGG CTCTTGTGCG 1560
TACTGTCCTT CGGGCTGGTG ACAGGGAAGA CATCACTGAG CCTGCCATCT GTGCTCTTCG 1620
TCATCTGACC AGCCGACACC AAGAAGCAGA GATGGCCCAG AATGCAGTTC GCCTTCACTA 1680
TGGACTACCA GTTGTGGTTA AGCTCTTACA CCCACCATCC CACTGGCCTC TGATAAAGGC 1740
TACTGTTGGA TTGATTCGAA ATCTTGCCCT TTGTCCCGCA AATCATGCAC CTTTGCGTGA 1800
GCAGGGTGCC ATTCCACGAC TAGTTCAGTT GCTTGTTCGT GCACATCAGG ATACCCAGCG 1860
CCGTACGTCC ATGGGTGGGA CACAGCAGCA ATTTGTGGAG GGGGTCCGCA TGGAAGAAAT 1920
AGTTGAAGGT TGTACCGGAG CCCTTCACAT CCTAGCTCGG GATGTTCACA ACCGAATTGT 1980
TATCAGAGGA CTAAATACCA TTCCATTGTT TGTGCAGCTG CTTTATTCTC CCATTGAAAA 2040
CATCCAAAGA GTAGCTGCAG GGGTCCTCTG TGAACTTGCT CAGGACAAGG AAGCTGCAGA 2100
AGCTATTGAA GCTGAGGGAG CCACAGCTCC TCTGACAGAG TTACTTCACT CTAGGAATGA 2160
AGGTGTGGCG ACATATGCAG CTGCTGTTTT GTTCCGAATG TCTGAGGACA AGCCACAAGA 2220
TTACAAGAAA CGGCTTTCAG TTGAGCTGAC CAGCTCTCTC TTCAGAACAG AGCCAATGGC 2280
TTGGAATGAG ACTGCTGATC TTGGACTTGA TATTGGTGCC CAGGGAGAAC CCCTTGGATA 2340
TCGCCAGGAT GATCCTAGCT ATCGTTCTTT TCACTCTGGT GGATATGGCC AGGATGCCTT 2400
GGGTATGGAC CCCATGATGG AACATGAGAT GGGTGGCCAC CACCCTGGTG CTGACTATCC 2460
AGTTGATGGG CTGCCAGATC TGGGGCATGC CCAGGACCTC ATGGATGGGC TGCCTCCAGG 2520
TGACAGCAAT CAGCTGGCCT GGTTTGATAC TGACCTGTAA ATCATCCTTT AGCTGTATTG 2580
TCTGAACTTG CATTGTGATT GGCCTGTAGA GTTGCTGAGA GGGCTCGAGG GGTGGGCTGG 2640
TATCTCAGAA AGTGCCTGAC ACACTAACCA AGCTGAGTTT CCTATGGGAA CAATTGAAGT 2700
AAACTTTTTG TTCTGGTCCT TTTTGGTCGA GGAGTAACAA TACAAATGGA TTTTGGGAGT 2760
GACTCAAGAA GTGAAGAATG CACAAGAATG GATCACAAGA TGGAATTTAG CAAACCCTAG 2820
CCTTGCTTGT TAAAATTTTT TTTTTTTTTT TTTTAAGAAT ATCTGTAATG GTACTGACTT 2880

TGCTTGCTTT GAAGTAGCTC TTTTTTTTTT TTTTTTTTTT TTTTTTTGCA GTAACTGTTT 2940

TTTAAGTCTC TCGTAGTGTT AAGTTATAGT GAATACTGCT ACAGCAATTT CTAATTTTTA 3000

AGAATTGAGT AATGGTGTAG AACACTAATT AATTCATAAT CACTCTAATT AATTGTAATC 3060

TGAATAAAGT GTAACAATTG TGTAGCCTTT TTGTATAAAA TAGACAAATA GAAAATGGTC 3120

CAATTAGTTT CCTTTTTAAT ATGCTTAAAA TAAGCAGGTG GATCTATTTC ATGTTTTTGA 3180

TCAAAAACTA TTTGGGATAT GTATGGGTAG GGTAAATCAG TAAGAGGTGT TATTTGGAAC 3240

CTTGTTTTGG ACAGTTTACC AGTTGCCTTT TATCCCAAAG TTGTTGTAAC CTGCTGTGAT 3300

ACGATGCTTC AAGAGAAAAT GCGGTTATAA AAAATGGTTC AGAATTAAAC TTTTAATTCA 3360

TT 3362